# EUROPEAN PATENT SPECIFICATION

(11) **EP 0 274 826 B1**
(45) Date of publication and mention of the grant of the patent: **12.08.1998**
(21) Application number: 87310029.1
(22) Date of filing: 12.11.1987
(51) Int. Cl.: C12N 15/12, C12Q 1/68, C12P 21/02, C07K 14/435, G01N 33/68, A61K 38/17

(54) **Recombinant Alzheimer's amyloid protein**
Rekombiniertes Alzheimer Amyloid-Protein
Protéine recombinante amyloide d'alzheimer

(30) Priority: 17.11.1986 US 932193; 31.12.1986 US 948376; 30.01.1987 US 8810; 18.08.1987 US 87002
(43) Date of publication of application: 20.07.1988
(73) Proprietor: SCIOS INC., Mountain View, CA 94043 (US)
(72) Inventor: Greenberg, Barry D., San Carlos California 94070 (US); Fuller, Forrest H,, San Carlos, California, (US); Ponte, Phyllis A,, Mountain View, California, (US)
(74) Representative: Goldin, Douglas Michael

(56) References cited:
- US-A- 4 666 829
- PROC. NATL. ACAD. SCI. USA, vol. 84, June 1987, pages 4190-4194; N.K. ROBAKIS et al.: "Molecular cloning and characterization of a cDNA encoding the cerebrovascular and the neuritic plaque amyloid peptides"
- NATURE, vol. 325, 19th February 1987, pages 733-736; J. KANG et al.: "The precursor of Alzheimer's disease amyloid A4 protein resembles a cell-surface receptor"
- SCIENCE, vol. 235, 20th February 1987, pages 877-880; D. GOLDGABER et al.: "Characterization and chromosomal localization of a cDNA encoding brain amyloid of Alzheimer's disease"
- BIOLOGICAL ABSTRACTS, vol. 83, no. 6, June 1987, abstract no. 56976, Philadelphia, PA, US; R.L. NEVE et al.: "A complementary DNA for a human microtubule associated protein 2 epitope in the Alzheimer neurofibrillary tangle", & MOL. BRAIN. RES. 1(2): 193-196, 1986
- SCIENCE, vol. 235, February 1987, pages 880-884; R.E. TANZI et al.: "Amyloid beta protein gene: cDNA, mRNA distribution, and genetic linkage near the Alzheimer locus"

## Description

### Technical Field

The invention relates to the diagnosis and treatment of Alzheimer's disease. More specifically, it relates to the use of materials related to amyloid protein deposits associated with Alzheimer's disease for diagnosis.

### Background Art

The demography of Alzheimer's disease is becoming progressively better understood. It is estimated that over 5% of the U.S. population over 65 and over 15% of the U.S. population over 85 are beset with this disease (Cross, A.J., Eur J Pharmacol (1982) 82:77-80; Terry, R.D. et al, Ann Neurol (1983) 14:497-506). It is believed that the principal cause for confinement of the elderly in long term care facilities is due to this disease, and approximately 65% of those dying in skilled nursing facilities suffer from it.

To confound the problem that therapy is at present a matter of experimentation, diagnosis is also unreliable. There is no straightforward diagnostic test, and diagnosis is made by a series of evaluations based on negative results for alternative explanations for the symptomologies exhibited. Assuming that the presence of the disease can be assessed accurately after death by autopsies of the brain, current results show that present diagnostic methods among living individuals carry an approximately 20% rate of false positives.

It would be extremely helpful in effecting appropriate care for patients and in developing therapies to have a straightforward assay method for diagnosing the presence of Alzheimer's disease. The invention described below provides an approach to this diagnosis.

Certain facts about the biochemical and metabolic phenomena associated with the presence of Alzheimer's disease are known. Two morphological and histopathological changes noted in Alzheimer's disease brains are neurofibrillary tangles (NFT) and amyloid deposits. Intraneuronal neurofibrillary tangles are present in other degenerative diseases as well, but the presence of amyloid deposits both in the interneuronal spaces (neuritic plaques) and in the surrounding microvasculature (vascular plaques) seems to be characteristic of Alzheimer's. Of these, the neuritic plaques seem to be the most characteristic (Price, D.L. et al, Drug Development Research (1985) 5:59-68).

The protein which makes up the bulk of these plaques has been partially purified and sequenced. Plaque-rich brains of deceased Alzheimer's patients have been used as a source to extract an approximately 4.2 kd "core" polypeptide, amyloid plaque core protein (APCP), herein referred to as "β-amyloid core protein." This peptide was designated B-protein by Glenner, G., et al, [Biochem Biophys Res Commun (1984) 120:885-890]. The amino acid sequence of the amino-terminus has been determined [Glenner. G., et al, Biochem Biophys Res Commun (1984) 122:1131-1135; Masters, C.L., et al, Proc Natl Acad Sci USA (1985) 82:4245-4259]. The amino acid sequences reported by the two groups above are identical except that Glenner et al, report a glutamine at position 11 for Alzheimer Disease cerebral vascular amyloid whereas Masters et al, report glutamic acid at position 11. Also, the former authors report that the cerebral vascular amyloid has a unique amino-terminus while the latter authors report that the form found in amyloid plaque cores has a "ragged" amino-terminus --i.e., peptides isolated from this source appear to be missing 3, 7, or 8 amino acids from the amino-terminus. Both groups have shown that the same peptide is found in the amyloid plaque cores and vascular amyloid of adult Downes syndrome-afflicted individuals and report glutamic acid at position 11.

Further studies on the β-amyloid core protein were also conducted by Roher, A. et al, Proc Natl Acad Sci USA (1986) 83:2662-2666 which showed the complete amino acid composition of the protein, and verified that it matched that of no known protein. The compositions obtained were, however, evidently not in agreement with those of Allsop, D., et al, Brain Res (1983) 259:348-352; nor were they in agreement with those published by Glenner or Masters (supra).

Wong, C.W. et al Proc Natl Acad Sci USA (1985) 82:8729-8732 showed that a synthetic peptide which was homologous to the first ten amino acids of the β-amyloid core protein described by Masters (supra) was able to raise antibodies in mice and that these antibodies could be used to stain not only amyloid-laden cerebral vessels, but neuritic plaques as well. These results were confirmed by Allsop, D. et al, Neuroscience Letters (1986) 68:252-256 using monoclonal antibodies directed against a synthetic peptide corresponding to amino acids 8-17. Thus, in general, the plaque protein found in various locations of the brain of Alzheimer's patients appears to be similar in immunoreactivity. It is highly insoluable, as shown by the inability to achieve solubilization in many commonly used denaturants such as detergents and chaotropic agents (Masters, supra, Allsop, D., et al, (supra)).

It is believed, by analogy to other amyloid proteins, that β-amyloid core protein may be formed from a precursor in the peripheral circulatory system or lymphatic system. There are six known instances of disease-associated amyloid deposits in which the nature of the precursor protein for the amyloid protein is known: for primary amyloidosis, the source is an immunoglobulin light chain; for secondary amyloidosis, the precursor is amyloid A protein; for familial amyloid polyneuropathy and senile cardiac amyloidosis, prealbumin or a variant thereof; for medullary carcinoma of thyroid, a procalcitonin fragment; and for hereditary cerebral hemorrhage, gamma-trace fragment (See, e.g., Glenner, G. New England Journal of Medicine (1980) 302:1283; Sletton, K. et al, Biochem J (1981) 195:561; Benditt, et al, FEBS Lett (1971) 19:169; Sletton, K., et al, Eur J Biochem (1974) 41:117; Sletton, K., et al, J Exp Med (1976) 143:993). The foregoing is a partial list and there are at least a number of additional references with regard to procalcitonin fragment as a precursor for the amyloid of the thyroid carcinoma. Alternatively, or additionally, such a precursor for β-amyloid core protein may be produced in the brain.

It has been described that a protein containing the β-amyloid core protein sequence within the framework of a larger protein exists (Kang, J et al, Nature (1987) 325:733-736). This protein, which is a potential precursor in vivo to the β-amyloid core protein, was predicted from the sequence of a cDNA clone isolated from a human fetal brain tissue cDNA library and consists of 695 amino acid residues wherein the amino terminus of the β-amyloid core protein begins at position 597. By analogy to the above described series, it may be that such a precursor or a fragment thereof circulates in the serum at a level differentiable in Alzheimer's victims relative to unafflicted individuals. Alternatively or additionally, such differences may be detected in the cerebral spinal fluid.

An alternative mechanism which could lead to the production of a β-amyloid core protein in vivo is suggested by the observation that the sequences encoding the first amino acid (Asp) of the β-amyloid core protein is directly proceeded in the genome by the codon for a methionine, which is the initiating amino acid for protein synthesis. Selection of this methionine by the translational apparatus of a cell as an initiator methionine, followed by its enzymatic removal by an aminopeptidase as frequently occurs in vivo, would give rise to a protein with the amino terminus of the β-amyloid core protein.

### Disclosure of the Invention

It is one general object of the invention to provide DNA sequence and protein compositions for β-amyloid-related proteins which can be used for improved screening, diagnosis, characterization, and study of the etiology of Alzheimer's disease.

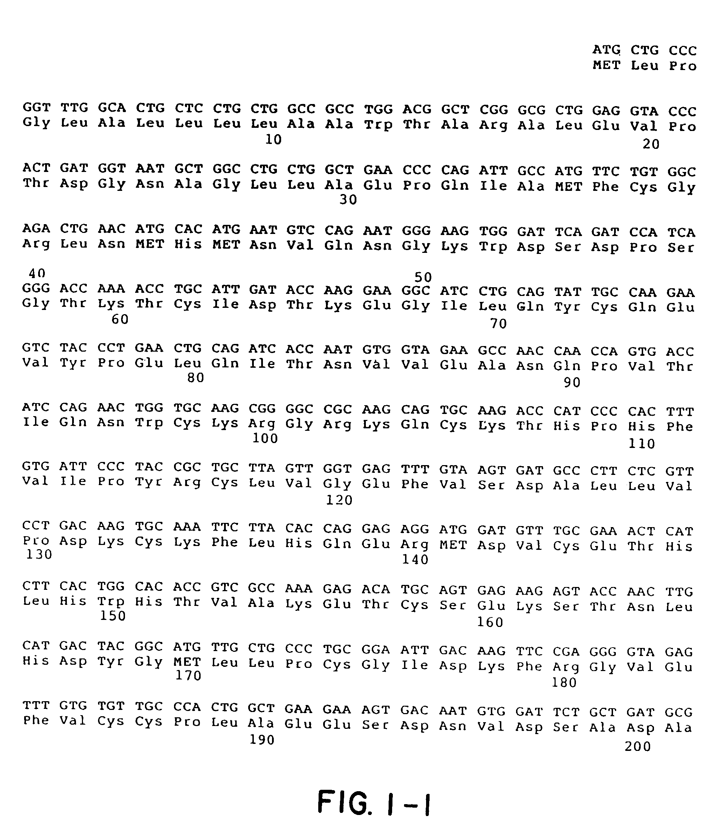

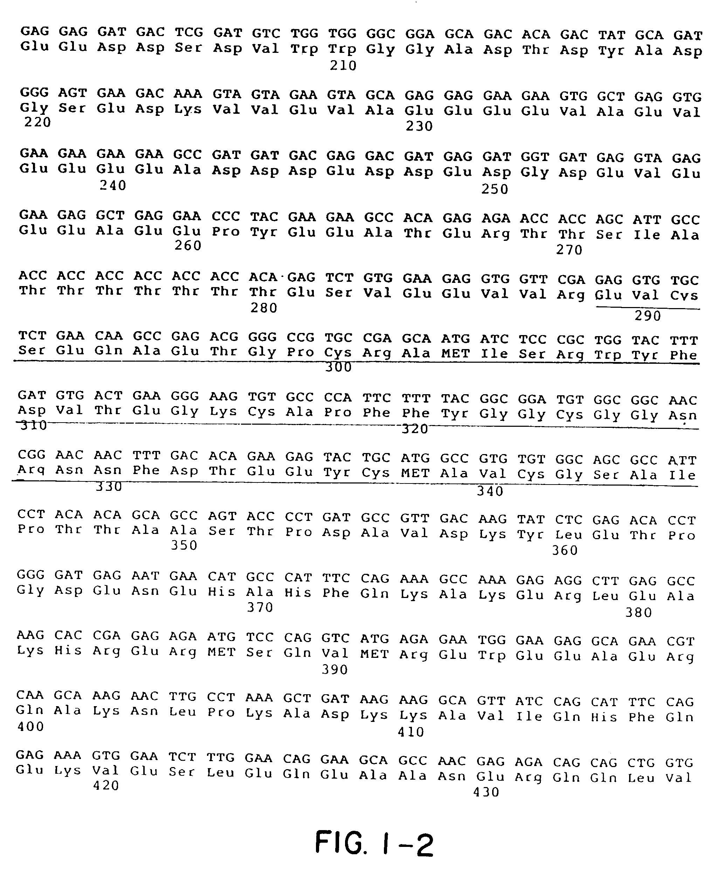

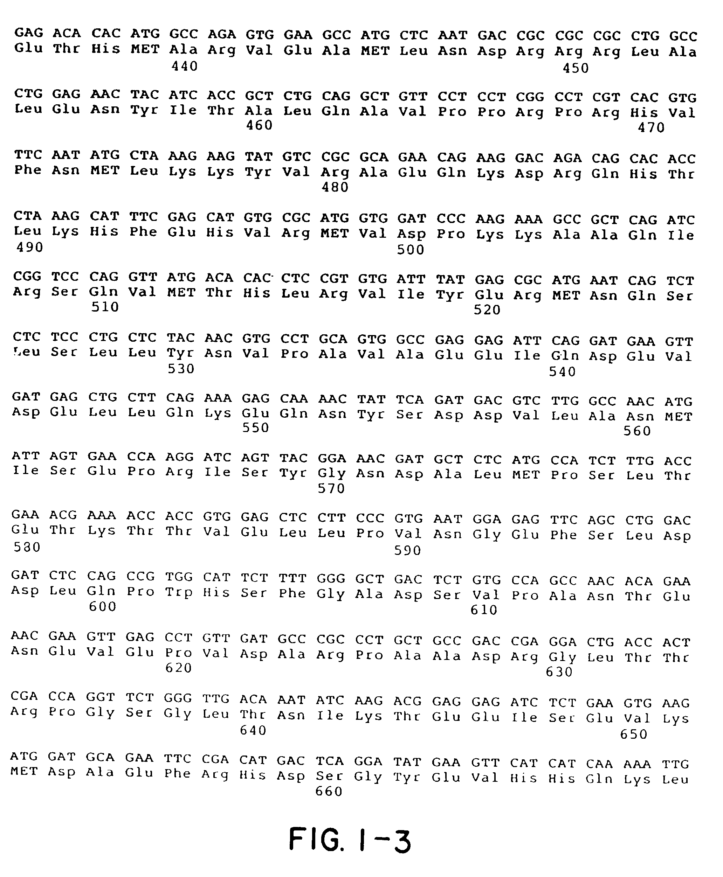

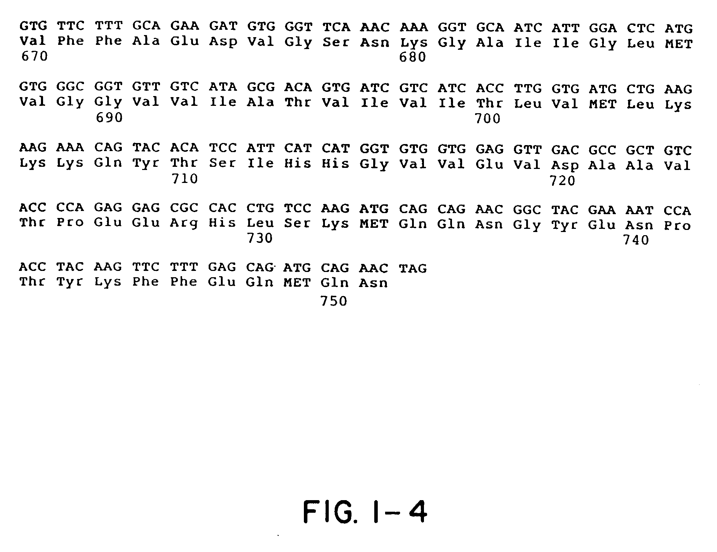

The invention provides DNA sequences useful in the prognosis and diagnosis of Alzheimer's disease in human subjects comprising the DNA sequence of Figure 1 or a subfragment thereof provided that such a subfragment does not consist of the DNA encoding the 28 amino-terminal amino acid residues of the β-amyloid core protein, or a subfragment thereof and such a subfragment contains the sequence encoding amino acid nos 289-345.

In a preferred embodiment of this aspect of the invention is provided a DNA sequence wherein a subfragment of the sequence shown in Figure 1 corresponds to the 168 basepair insert fragment of the β-amyloid-related gene product of bacteriophage λAPCP16814.

In yet another aspect of the invention, recombinant β-amyloid-related proteins obtained by the expression of the above-described DNA sequences are provided.

A further aspect of the invention relates to a method of diagnosing a genetic predisposition to Alzheimer's disease in a test subject, comprising identifying, as being associated with predisposition to Alzheimer's disease, one or more alterations in the afore-described DNA, and assaying test subject gene fragments for the presence or absence of such alteration(s).

A related prognostic test provides a method of diagnosing a genetic predisposition to Alzheimer's disease in a test subject, comprising identifying, as being associated with a predisposition to Alzheimer's disease, one or more restriction site alterations in the DNA sequences of Figures 1, and assaying test subject gene fragments in vitro for the presence or absence of such restriction site alteration(s).

A further embodiment provides a method of screening for Alzheimer's disease in a test subject, comprising preparing a peptide which includes an immunogenic region of the protein of the invention, eliciting antibodies which are specific against peptide, and using the antibodies to detect the increase or decrease of β-amyloid-related proteins in a test subject suspected of having Alzheimer's disease.

Yet a further embodiment of the invention relates to the use of a polypeptide of the sequence 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 for the manufacture of a composition useful for treating Alzheimer's disease.

These and other objects and features of the invention will become more fully apparent when the following detailed description of the invention is read in conjunction with the accompanying drawings.

### Brief Description of the Drawings

Figure 1 shows the base sequence of a cDNA clone, designated λAPCP168i4, which encodes amino acids 1-751 of β-amyloid-related protein. The 168 bp insert, which distinguishes this clone from the Kang et al sequence, is underlined.
Figure 2 shows a DNA sequence of a genomic clone encoding the first 18 amino acids of the β-amyloid core protein as described by Masters et al. It also encodes, immediately preceding these amino acids, a methionine codon which could potentially be used as an initiating codon;
Figure 3 shows the base sequence of a cDNA clone, designated XSM2W4, whose 3' end encodes the first four amino acids of β-amyloid core protein. It also encodes, immediately preceding these amino acids, a methionine codon as described above;
Figure 4 shows the base sequence of a cDNA clone, designated λSM2W3, which encodes 97 amino acids; the first 26 of these correspond to the region of the β-amyloid core protein described by Masters et al, from Glu₃ through Ala₂₈;
Figure 5 shows the base sequence and corresponding amino acid sequence of a β-amyloid-related protein deduced from λSM2W4 and λSM2W3;
Figure 6 shows the nucleotide and deduced amino acid sequence of the λSMW9 β-amyloid clone;
Figure 7 shows a comparison of the sequences of λSM2W3 and λSM2W9;
Figure 8 shows the detection of mRNAs for λAPCP168i4 and the mRNA described by Kang et al on a Northern blot produced using RNA's isolated from human brain and human cells in culture and hybridized to oligonucleotide probes which are specific for each species;
Figure 9 shows the construction scheme for a bacterial expression vector for the production of a β-amyloid-related protein in bacteria;
Figure 10 shows the construction scheme for a recombinant vaccinia virus expression vector for the expression of the protein encoded by λAPCP168i4;

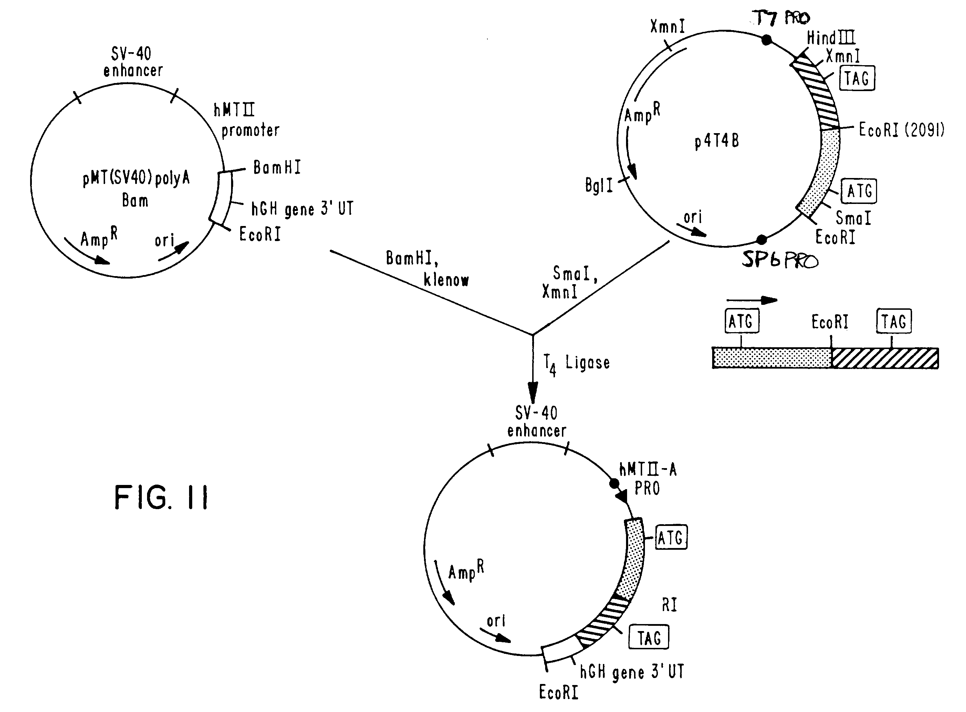

Figure 11 shows the construction scheme for a mammalian cell expression vector for the expression of the protein encoded by λAPCP168i4;
Figure 12 shows the construction of an expression vector for the production of the β-amyloid-related protein described in Figure 5, when the methionine encoded immediately upstream from the β-amyloid core protein sequence is used as an initiating methionine;
Figure 13 shows the relatedness of the peptide encoded by the λAPCP168i4 168 bp insert to a superfamily of proteins many of whose members exhibit inhibitory activity for basic proteases; and
Figure 14 shows the construction of a synthetic tryptophan operon promoter and operator regulatory sequence, and a restriction site map of plasmid pTRP233.

### Detailed Description of the Invention

### A. Definitions

As used herein, "β-amyloid core protein" means the protein described by Masters, C.L., et al Proc Natl Acad Sci USA (1985) 82:4245-4249, herein referred to as "Masters, et al". This approximately 4 kD protein is defined at the amino terminus by sequence analysis as a mixture of four peptides with slightly different amino termini, the amino termini of the three smaller peptides being completely encoded by that of the largest. The first 28 amino acids of the longest form is
Asp₁-Ala₂-Glu₃-Phe₄-Arg₅-His₆-Asp₇-Ser₈-Gly₉- Tyr₁₀-Glu₁₁-Val₁₂-His₁₃-His₁₄-Gln₁₅-Lys₁₆-Leu₁₇-Val₁₈-Phe₁₉-Phe₂₀-Ala₂₁-Glu₂₂-Asp₂₃-Val₂₄-Gly₂₅-Ser₂₆-Ser₂₇-Ala₂₈. The rest of the molecule is undefined by sequence analysis.

"β-amyloid-related protein or "β-amyloid-related peptide" are defined herein as those proteins containing within their sequence the β-amyloid core protein sequence defined above or fragments of such proteins which do not necessarily include the β-amyloid core protein sequence as defined above. As an example, this term is used to refer to the protein described by Kang, J. et al, Nature (1987) 325:733-736, herein referred to as "Kang, et al" which contains the β-amyloid core protein within its structure at amino acid 597 of a 695 amino acid protein. As another example, it refers to the protein encoded by λAPCP168i4, shown in Figure 1, which contains the β-amyloid core protein within its structure at amino acid 653 of a 751 amino acid protein.

"Immunogenic β-amyloid core peptide" or "immunogenic β-amyloid-related peptide" refer to peptides whose amino acid sequences match those of some region of the β-amyloid core protein or β-amyloid-related protein, and which are capable of provoking an antibody response in an immunized animal.

"Genetic predisposition to Alzheimer's disease" refers to an identifiable genetic mutation or alteration found in the genomes of individual's with Alzheimer's disease, or those individuals destined to develop Alzheimer's disease, but not normal (nondiseased) individuals.

### B. DNA Sequences

DNAs corresponding to β-amyloid core protein or β-amyloid-related protein sequences are useful as probes in diagnosis. Several DNAs containing sequences encoding portions of β-amyloid-related protein sequence, and β-amyloid core protein sequence with adjacent noncoding segments are disclosed herein. These DNA sequences in whole or in part, are thus useful in diagnosis, either as intact probes, or as fragments.

In particular, the invention includes a DNA sequence which encodes a β-amyloid-related protein comprising the nucleotide sequence and corresponding, deduced amino acid sequence set forth in Figure 1. This DNA sequence encodes an approximately 82,610 dalton protein containing the β-amyloid-related core protein.

The present β-amyloid protein cDNA sequence, set forth in Figure 1, can be isolated from bacteriophage λAPCP168i4. This human fibroblast cDNA clone was obtained from a cDNA library prepared in λgt10 using standard techniques from SV40-transformed fibroblast (SV80) cells (Todaro, G.J. et al, Science (1966) 153:1252-1254). The λgt10-SV80 library was screened with a mixture of labelled oligonucleotides. Two unique phage containing β-amyloid-related sequences were obtained; these β-amyloid-related sequences were subcloned into a plasmid vector and sequencing analysis revealed a sequence co-linear with the sequence encoding the Kang et al β-amyloid-related protein, except for the presence of a 168 basepair insert. The 168 basepair insert interrupts the codon for Val₂₈₉ of the Kang et al sequence, resulting in the loss of this amino acid from the λAPCP168i4 protein. The 168 basepair insert, together with the 3 basepairs gained from the interrupted Val₂₈₉ codon, encode 57 new amino acids, which are underlined in Figure 1. Downstream of this insertion, at codon 653 of Figure 1, lies the amino-terminal aspartate of the β-amyloid core protein described by Masters et al. The λAPCP168i4 clone was deposited at ATCC on 1 July 1987 under the accession number 40347.

Particularly useful are those sequences which encode the 57 amino acid insert found in λAPCP168i4, as well as sequences encoding the corresponding "junction" of the Kang et al β-amyloid-related protein sequence.

For example, one preferred embodiment comprises DNA sequences encoding a β-amyloid-related protein having an amino acid sequence corresponding to residues 289 through 345 of the above-identified protein. Thus, this embodiment comprises a β-amyloid-related protein of the amino acid sequence:

Glu Val Cys Ser Glu Gln Ala Glu Thr Gly Pro Cys Arg Ala
                                    10

MET Ile Ser Arg Trp Tyr Phe Asp Val Thr Glu Gly Lys Cys
                    20

Ala Pro Phe Phe Tyr Gly Gly Cys Gly Gly Asn Arg Asn Asn
     30                                      40

Phe Asp Thr Glu Glu Tyr Cys MET Ala Val Cys Gly Ser Ala
                             50

Ile.

This particular peptide, including any fragments thereof, distinguishes the present β-amyloid-related protein from other reported forms.

In another preferred embodiment, the invention discloses a β-amyloid-related protein having the DNA sequence and deduced amino acid sequence corresponding to amino acid residues 284-Val₂₈₉ -(∇289-345)-349 of the β-amyloid-related sequence set forth in Figure 1 (wherein ∇ symbolizes a deletion of residues 289 through 345). An oligopeptide spanning this specific region would be useful to generate a protein specific diagnostic reagent to differentiate between the β-amyloid-related protein genetic variant described by Kang et al and the β-amyloid-related protein of the present invention. Thus, this embodiment comprises a β-amyloid-related protein of the amino acid sequence: 
Glu Glu Val Val Arg Val Pro Thr Thr Ala
                5                   10
 A smaller peptide contained within the sequence of this peptide might also be used.

Oligonucleotides specific for the 168 basepair insert and for the junctions of this region of the β-amyloid-related protein described by Kang et al can be synthesized and used to compare the levels of mRNA expression of these two distinct proteins. As an example, oligonucleotides specific for the 168 basepair insert, designated oligo #2734 
10 20 30 40 50
(CGCCGTAAAA GAATGGGGCA CACTTCCCTT CAGTCACATC AAAGTACCAG

60
CGGGAGATCA)
 and for the "junction" region, designated oligo #2733 
10 20 30
(CTGCTGTTGT AGGAACTCGA ACCACCTCTT)
 were synthesized using phosphoramidite chemistry on an Applied Biosystems DNA synthesizer.

The "junction" oligo is complementary to 15 basepairs on either side of the insert and is used to distinguish between the published β-amyloid-related protein sequences and the λAPCP168i4 sequences by specific hybridization conditions known in the art under which a 15 basepair perfect match is unstable, while a 30 basepair perfect match is stable. These oligonucleotides are used to screen cDNA libraries or mRNA from various sources as an assay for measuring the level of expression of a specific sequence.

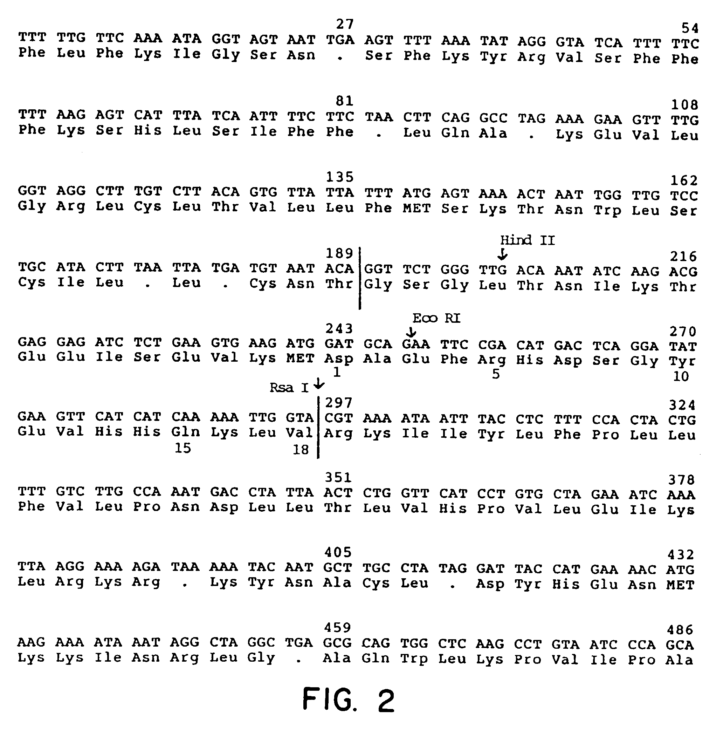

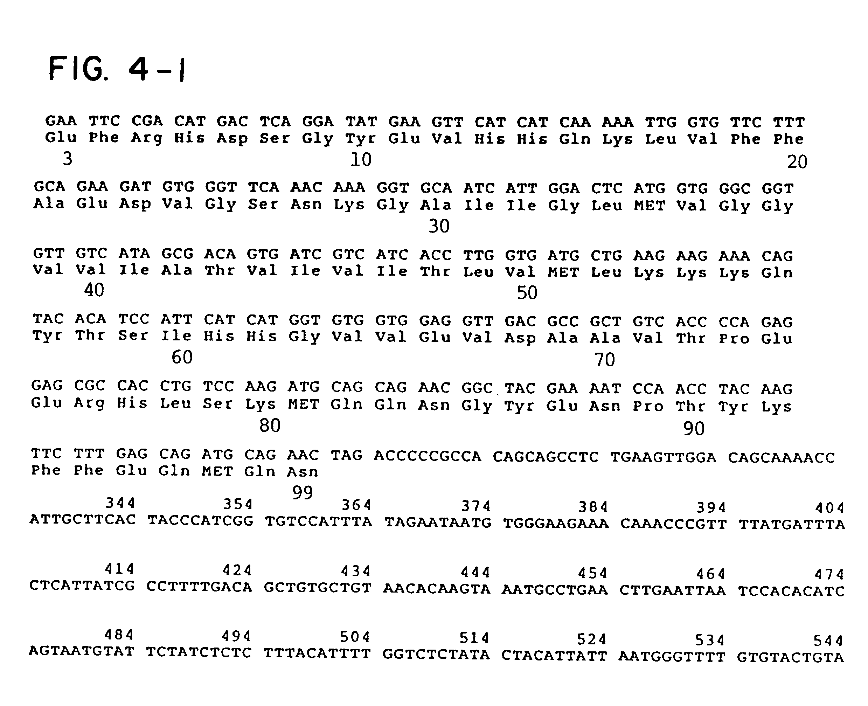

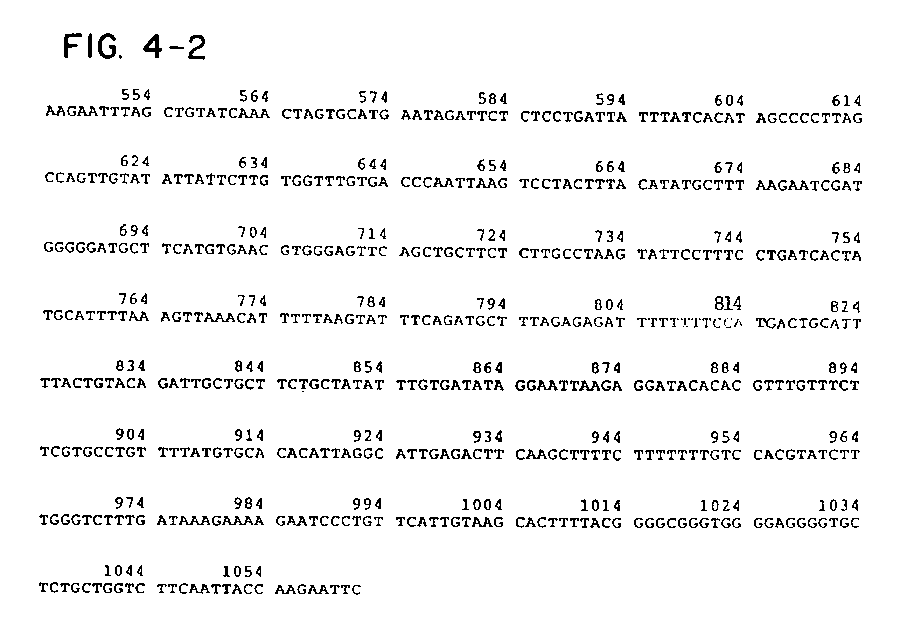

Another example of DNA encoding a portion of the β-amyloid protein, described below, is a genomic sequence encoding the first 18 amino acids (19 if met is included) of the β-amyloid protein sequence characteristic of Alzheimer's disease in neuritic plaques. The clone was obtained in λ Charon 4A from the genomic library described by Lawn, R.M., et al, Cell (1978) 15:1157-1174 and has been partially sequenced, as shown in Figure 2. As seen, the sequenced portion of the genomic clone includes a 57 base pair segment which encodes the amino acids 1-18 of the previously reported β-amyloid core protein and a methionine immediately preceeding. Downstream of the amino acid 18 codon, the genomic sequence diverges in codon sequence from that expected from the reported amino acid sequence of β-amyloid core protein. By reference to the protein encoded by the sequence of Figure 4, and by inspection of the sequences flanking this region using knowledge known in the art, this divergence is likely to be an intron sequence. This clone, designated λSM2, was deposited at ATCC on 13 November 1986.

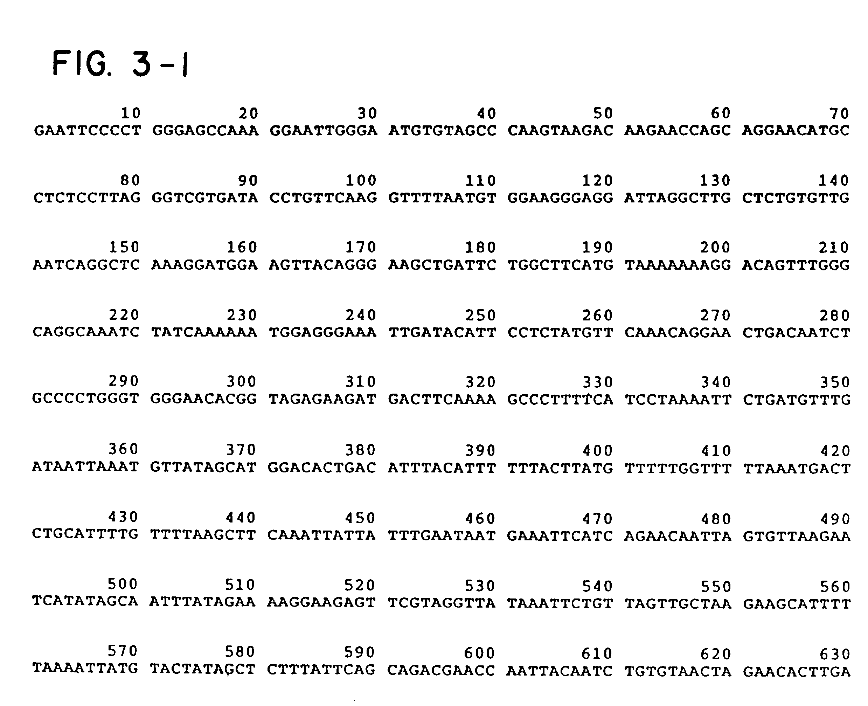

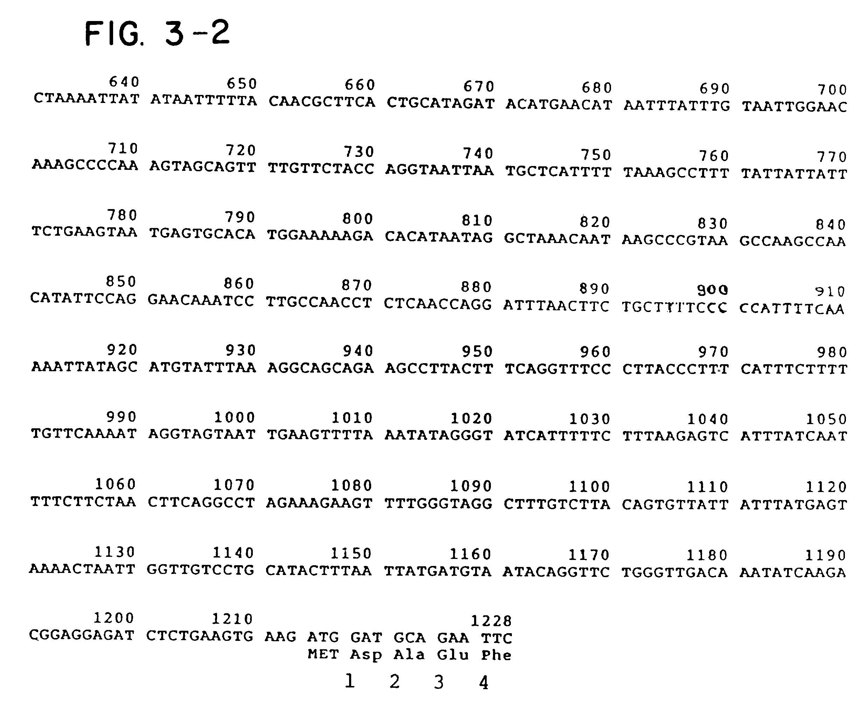

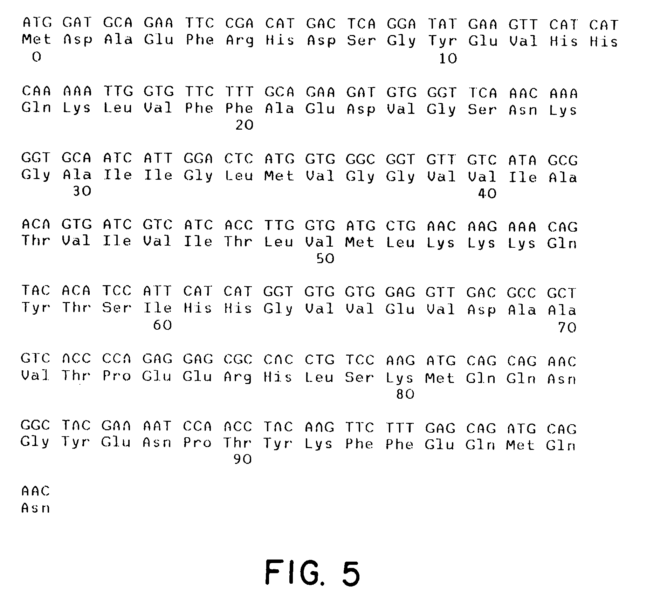

A HindIII/RsaI probe derived from the genomic clone (see Figure 2) was used as a probe to isolate, according to standard procedures, cDNA fragments from a cDNA library constructed in λgt10 from temporal and parietal cortical tissue of a normal human brain (the individual was a 55 year old man who died of myocardial infarction). The three cDNA clones which were isolated were sequenced conventionally, and matched with amino acid sequences in each of the three possible reading frames to identify regions coding for β-amyloid-related proteins. One of the clones, designated λSM2W4, contains a 3'-end terminal sequence which encodes the Asp Ala Glu Phe amino acids at the 5'-end of β-amyloid-core protein, as seen in Figure 3, which shows the complete base sequence of the clone. The Asp₁ codon is immediately preceeded by a methionine codon. A second clone, designated λSM2W3, contains a 5' region segment which has a 6 bp overlap with the 3' end of the λSM2W4 clone (an EcoRI restriction site), encoding amino acids 3 and 4 of the β-amyloid core protein, and an additional 95 codons which encode the remainder of a β-amyloid-related protein. The DNA sequence for the 100 amino acid protein (including Met) encoded in λSM2W4 and λSM2W3 is shown in Figure 5. It is, of course, understood that the methionine is probably processed in vivo, and that the β-amyloid-related protein represented in this figure may thus be a 99 amino acid sequence.

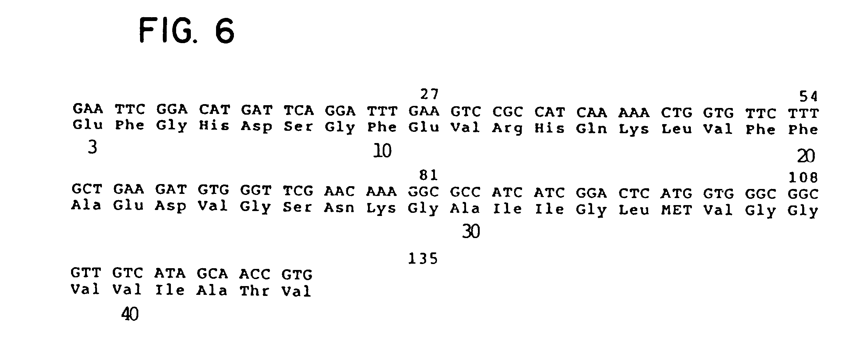

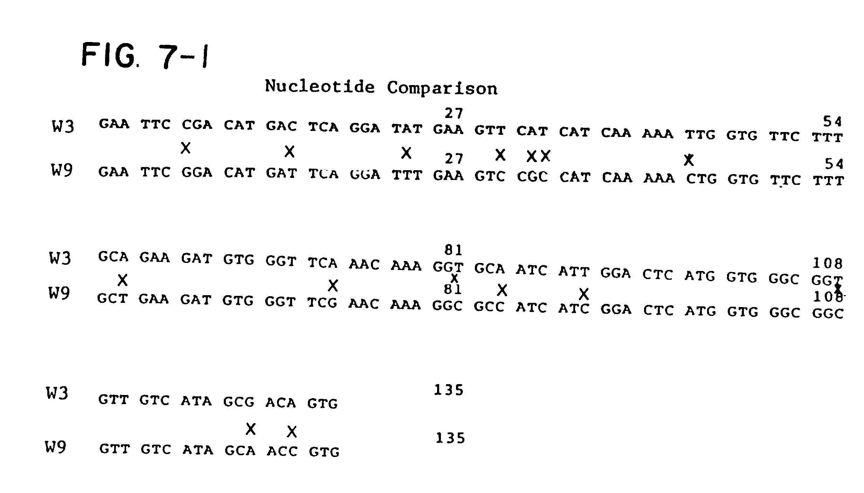

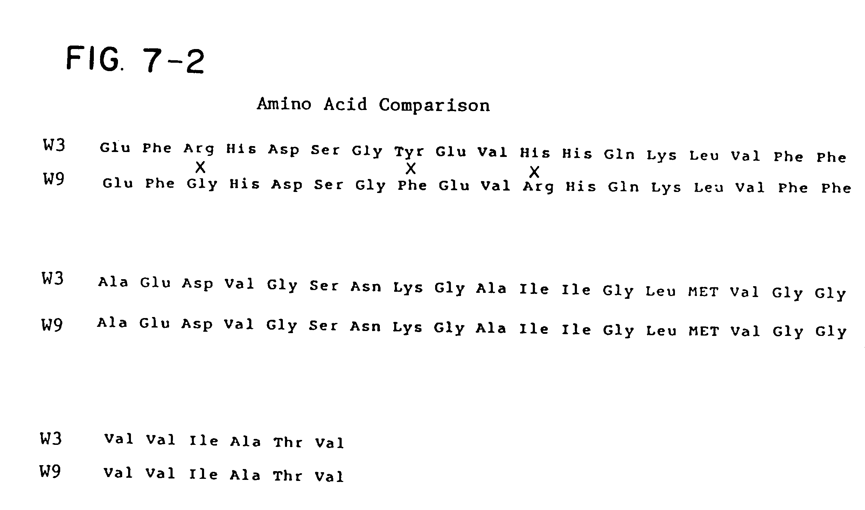

A third cDNA clone encodes a portion of a β-amyloid-related protein which differs from λSM2W3 in the region shown by 15 nucleotide differences and 4 amino acid differences in the region of amino acids 3-44 of Figure 5. The DNA sequence and deduced amino acid sequence for this clone, designated XSM2W9 are given in Figure 6. A comparison with λSM2W3 is given in Figure 7.

The λSM2W4, λSM2W3, λSM2W9, and λAPCP168i4 clones have been deposited with the American Type Culture Collection, Rock Lawn, MD and have ATCC Nos. 40299, 40300, 40304 and 40347, respectively.

### C. Protein Production

The four cDNA clones above permit construction of coding sequences which may be expressed to obtain a complete β-amyloid-related protein, an 100 amino acid β-amyloid-related protein containing the amino-terminal sequences reported for β-amyloid core protein, and other desired proteins. These sequences can be inserted in a suitable expression vector for production of protein. Details of the method of constructing a DNA subsequence of Figure 1 and insertion of this sequence into a bacterial expression vector is provided in Example 2.

Briefly, an E. coli expression vector, designated pAPCP118-3, was constructed for the expression of a fusion protein consisting of amino acid residues 655 to 751 set forth in Figure 1. The construction of pAPCP118-3 was accomplished by joining the following three fragments: (1) a plasmid backbone (consisting of pBR322 replication functions, an ampicillin resistance gene, the tryptophan promoter and operator, a ribosome binding site, DNA encoding the seven amino terminal codons of the beta-galactosidase structural gene followed by six threonine residues, and transcription termination signals); (2) an EcoRI-HaeII fragment encoding amino acid residues 655-728 of the Figure 1 sequence; and (3) a synthetic fragment encoding amino acid residues 729-751 of the Figure 1 sequence, followed by a stop codon.

The resulting vector was used to transform E. coli W3110 and expression of the fusion protein was induced by reducing the tryptophan concentration followed by the addition of 3-beta-indoleacrylic acid. The resulting protein can be purified using conventional purification techniques and the resulting purified material is available for use in the production of antibodies for diagnostic assays.

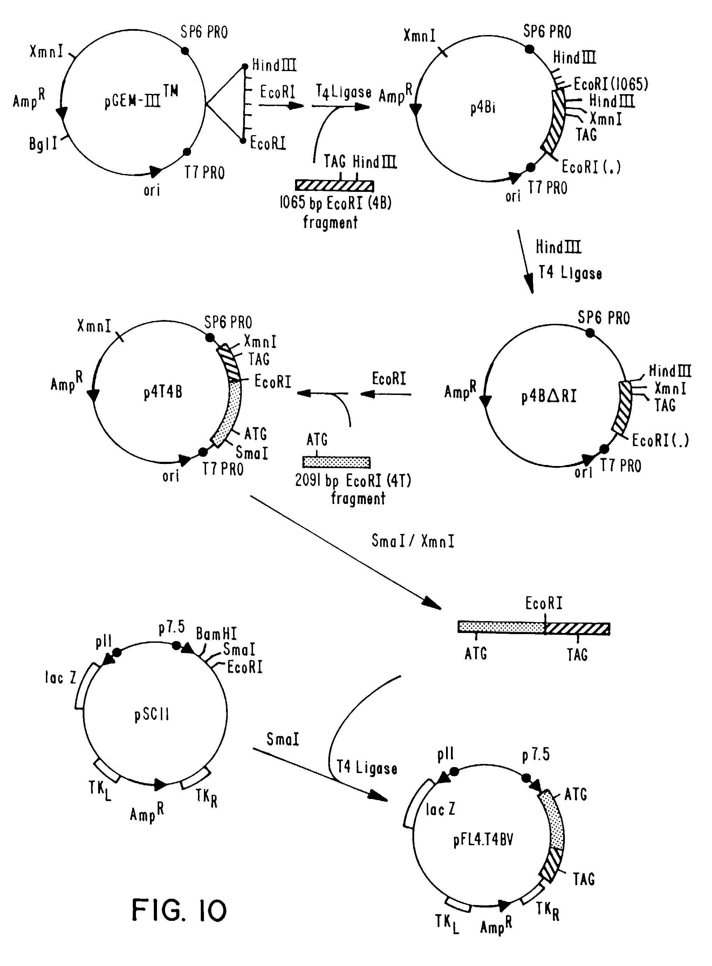

The complete coding sequence of the β-amyloid-related protein set forth in Figure 1 was subcloned in two fragments from the deposited λAPCP168i4 clone and inserted into pSC11, a vaccinia virus expression vector. The construction of the resulting vector, pFL4T4BV, is illustrated in Figure 10. Briefly, an approximately 1.06 kilobase (kb) EcoRI fragment, spanning amino acid residues 655-751 of the protein illustrated in Figure 1, was cloned into EcoRI-digested plasmid pGEM-3™ (available from Promega Biotec) to create an intermediate vector designated p4BI. Subsequently p4BI was digested with HindIII to remove much of the 3'-noncoding sequence of the β-amyloid-related sequence. The resulting vector p4BΔRI was digested with EcoRI and treated with calf intestinal alkaline phosphatase prior to ligation to the 2088 bp EcoRI fragment derived from λAPCP168i4 to form p4T4B. This plasmid was digested with SmaI and XmnI to generate a 2678 bp fragment spanning the complete protein encoding sequence set forth in Figure 1.

The gene encoded by this SmaI-XmnI fragment was inserted into a well-known vaccinia viral vector, pSC11, for subsequent expression of the β-amyloid-related protein in CV-1 monkey kidney cells using a eucaryotic transient expression system as described by Cochran, M.A., et al (1985) Proc Natl Acad Sci USA 82: 19-23. More commonly, this vector is used for in vivo protein and antibody production in animals after its sequences have been inserted into the vaccinia virus genome (see "Antibody Production" section below).

Similarly, mammalian vectors can be utilized for expression of the β-amyloid core protein or β-amyloid-related proteins described herein. For example, plasmid phGH-SV (10) (a plasmid described in EPA 217,822, published 15 April 1987,) contains a pUC8 plasmid backbone, hMT-IIa gene promoter and regulator elements, SV-40 DNA promoter and enhancer elements, and the coding portions of the hGH gene and 3' regulatory sequences. This plasmid can be digested with BamHI and SmaI and treated with BamHI linkers to delete the human growth hormone protein encoding sequence and leaving the 3'-noncoding sequences and regulatory elements attached to the plasmid backbone. This approximately 5100 base pair DNA piece is gel purified and ligated to BamHI linkers. Digestion with BamHI, repurification of the DNA fragment and subsequent ligation result in a plasmid designated pMTSV40 polyA Bam which contains the structural and regulatory elements comprising a mammalian cell expression vector. After BamHI digestion of pMTSV40 polyA Bam and repair in the presence of DNA polymerase I and all four dNTPs, this vector is available for, insertion of the ∼ 2678 bp SmaI- XmnI restriction fragment of plasmid p4T4B. The resulting vector can then be used for efficient protein expression in CHO cells as described in Example 4.

In addition, the sequence information from the λSM2W4 clone, illustrated in Figure 3, combined with the sequences present in the λSM2W3 clone, may be used to construct a mammalian cell expression vector encoding the protein described in Figure 5.

In the cases of protein production described above, the transformed cells are screened for production of the resulting β-amyloid-related protein using anti-β-amyloid antibody prepared as described below.

### D. Antibody Preparation

Antibodies specific against β-amyloid core protein and β-amyloid-related proteins are prepared by known procedures. As an example using synthetic peptides, typically the protein sequence is analysed for regions of at least about 10 amino acids long which have predominantly polar and/or charged amino acid residues to identify favorable immunogenic regions.

As another example, the DNA sequence shown in Figure 1 can be used to design oligopeptides which are specific to the inserted sequence in λAPCP168i4, as well as the corresponding junction of this insert to the β-amyloid-related protein described by Kang et al. For example, an oligopeptide spanning the inserted junction such as Glu-Glu-Val-Val-Arg-Val-Pro-Thr-Thr-Ala may be used to immunize animals to produce a specific antisera against this region of the protein described by Kang et al. Inspection of the Kang et al sequence in the absence of knowledge of the λAPCP168i4 sequence, would not provide the information necessary to identify this peptide as a valuable reagent by any method known in the art. As another example, oligopeptides designed to represent sequences present in the 168 basepair insert region could be used in a similar manner to generate antisera against this unique region of the APCP168i4 protein. Thus, the regions identified as favorable for immunogenicity are synthesized by conventional peptide synthetic methods, and coupled covalently to a suitable carrier protein, such as keyhole limpit hemocyanin. Antibodies are raised against the peptide/protein conjugate in rabbits or the like by conventional methods. The presence of antibody in immunized animals is detected by standard methods, such as immunoreactivity to the immunizing synthetic peptide affixed to a microtiter plate, followed by ELISA.

Another method of antibody production uses the bacterially produced β-amyloid-related fusion protein of example 2 as the immunogen. The immunogenicity of this protein is shown by the immunoreactivity of the antisera to the bacterially produced fusion protein.

Yet another method of antibody production relies on the inoculation of the host animal with a live recombinant vaccinia virus encoding β-amyloid-related protein, such recombinant viruses being generated by established techniques involving recombination between wild-type vaccinia virus and the vectors derived from pSC11, such as pFL4T4BV, described herein. These antibodies can then be used in the diagnostic assays described below.

A panel of antibodies which are specific against peptides derived from different regions of the β-amyloid-related protein, such as the 57 amino acid insert of λAPCP168i4, are further analysed for immunoreactivity of β-amyloid-related proteins present in the serum or cerebral spinal fluid of patients with Alzheimer's disease, to identify antibodies suitable for a diagnostic assay for Alzheimer's disease, as discussed below.

### E. Diagnostic and Prognostic Methods

The DNA sequences described in Figures 3, 4, and 6 for β-amyloid-related protein are primarily derived from an apparently normal advanced-age male showing no signs of Alzheimer's disease at the time of death. The λAPCP168i4 sequence described in Figure 1 for another β-amyloid-related protein is derived from cultured fibroblast cells. These sequences provide a standard for identifying mutations in genomic sequences which are found in individuals with Alzheimer's disease, and which are therefore likely to be associated with a predisposition to the disease.

### 1. Prognostic Methods.

Assays are used to determine an individual's genetic predisposition to Alzheimer's disease. These tests use the DNA sequences of the present invention in a comparative study with samples of the patient's DNA to define polymorphisms in the region of the chromosome containing the β-amyloid gene. Alternatively or concurrently, the DNA sequences of the present invention can be used in nucleic acid hybridization analysis to define alterations, which alterations are meant to include additions, deletions, mutations or substitutions, in the DNA or RNA encoding β-amyloid-related proteins.

Alterations in the β-amyloid-related protein sequences which correlate with Alzheimer's disease can be assayed by a differential probe binding method. Under appropriate hybridization conditions, known in the art, the oligonucleotide probes will bind to completely complementary sequences, but not to closely related but altered sequences.

In one assay method, nucleic acid samples prepared from the test subject are hybridized with each probe, under the defined hybridization conditions, and examined for binding to specific oligonucleotides. Alterations, and thus predisposition to Alzheimer disease, are confirmed by binding one probe, but not to the other probe. The probe-binding method, as it has been applied to other genetic diseases, is described in Conner, B.J., et al, Proc Nat Acad Sci (USA) 80:278-282 (1983).

Alternatively, probes derived from the genomic or cDNA sequences described above may be used to identify restriction fragment length polymorphisms which are associated with a genetic predisposition to Alzheimer's disease. Initially the probes are used to identify restriction site fragment lengths from both normal and diseased genomic digest samples. Changes in restriction fragment lengths which correlate with Alzheimer's disease are then applied to genetic screening, by standard methods. That is, test subject genomic material is digested with the restriction enzyme(s) of interest, and the fragment pattern on Southern blotting is determined with the labeled probe.

### 2. Diagnostic Methods.

In various other clinical amyloidoses, the amyloidogenic peptides are variants of normally expressed gene products. These peptides have been altered either by aberrant proteolytic processing or by genetic lesions yielding an alteration in the primary amino acid sequences. There are known amyloidosis, such as Familial Amyloid Polyneuropathy (FAP), in which a mixture of the normal precursor and the amyloidogenic variant coexist within the circulation. An aberrant tissue-distribution for the expression of the aberrant gene product, or some other alteration in its level of expression, its sequence, or its processing in Alzheimer's disease could have significance in terms of the etiology of amyloid deposition.

A first diagnostic test which utilizes the materials of the invention is a direct antibody assay for the increase or decrease of β-amyloid core protein or β-amyloid-related proteins in Alzheimer's individuals relative to normal individuals. In this method, antibodies obtained as described above are screened for specific immunoreactivity with proteins from individuals known to have Alzheimer's disease. The presence of immunoreactive serum proteins is determined by standard immunoassay techniques, such as solid-phase ELISA techniques.

The body sample which is assayed for the presence of β-amyloid core protein or β-amyloid-related protein is, for example, serum or cerebral spinal fluid. For instance, in hereditary cerebral hemorrhage with amyloidosis, a disorder wherein the amyloid is generated from the gamma-trace precursor, the precursor can be detected in cerebrospinal fluid using an immunoassay. The levels of the precursor are reduced in the patients having the disease, leading to the conclusion that it is used up during the formation of the deposits. The precursor is made in the brain, and hence the cerebrospinal fluid is the appropriate sample.

In another diagnostic test, DNA encoding β-amyloid-related protein is directly useful as a probe to detect an increase or decrease in synthesis of mRNAs encoding β-amyloid-related proteins in the appropriate target cells by virtue of its ability to hybridize to the appropriate mRNA. An example showing the utility of this method is given in Example 5 below.

A third diagnostic assay permits the detection of antibodies against the amyloid protein in patient's serum using such standard ELISA techniques wherein the purified recombinant amyloid protein or synthetic peptide is bound to the solid support.

### F. Therapeutic Methods.

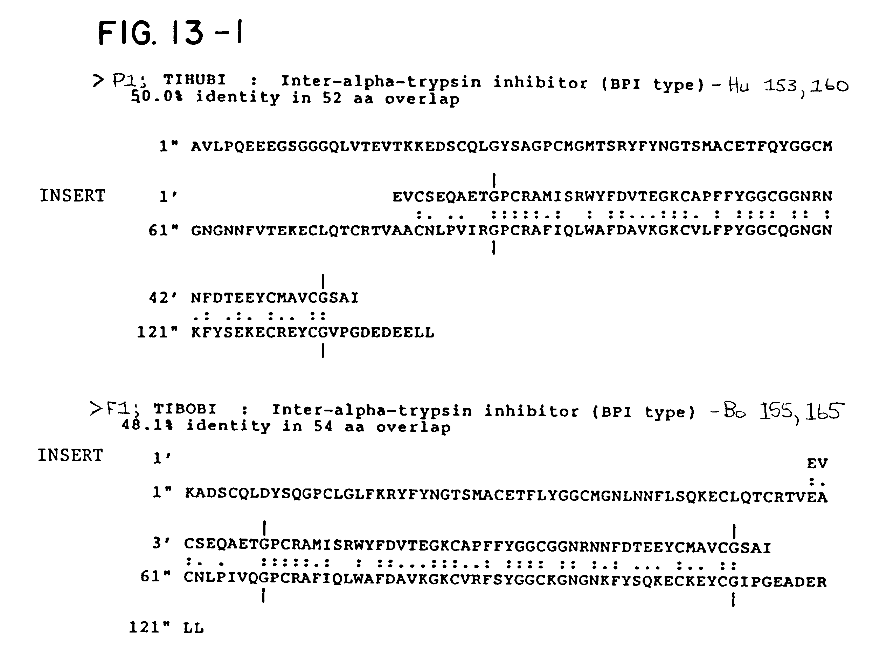

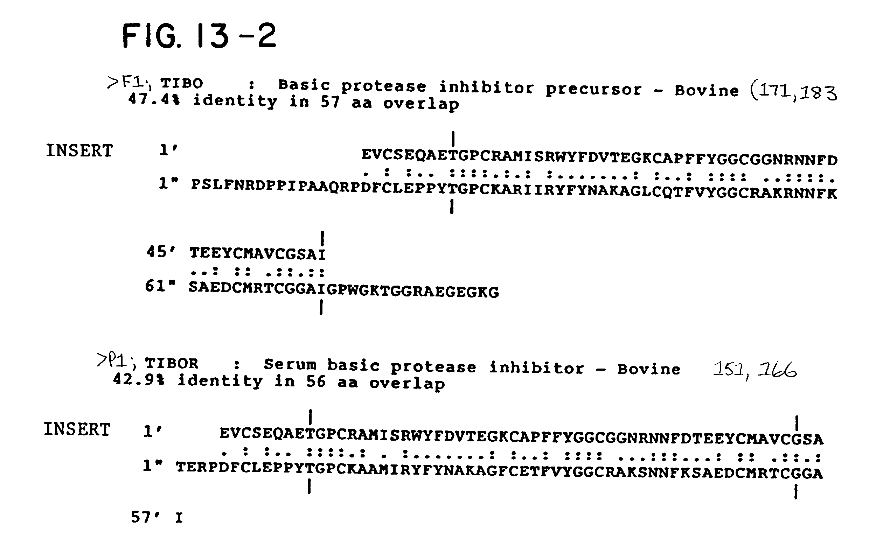

The invention also provides for improved therapeutic treatments for Alzheimer's disease. One therapeutic treatment is suggested by the sequence of the protein encoded by the 168 bp insert in λAPCP168i4. Using methods well known in the art such as the use of computer programs which search protein databases, to compare the protein relatedness of one protein to another, the protein encoded by the 168 bp insert was found to be highly homologous to a family of proteins known as Kunitz basic protease inhibitors. The level of relatedness of the insert protein segment to three members of the family is shown in Figure 13, where the symbol (:) indicates an identity between the two sequences compared and the symbol (.) indicates the substitution of an amino acid with similar chemical properties. The insert sequence, depicted by the one-letter amino acid code as EVCS ... GSAI is shown to be related to a high degree over its entire length to all members of the family (only three are shown as an example). The comparisons shown are to: (1) a human trypsin inhibitor, a secreted plasma protein which inhibits trypsin, plasmin and lysosomal granulocytic elastase (Wachter, E., and Hochstrasser, K. (1981) Hoppe-Seyler's Z Physiol Chem 362:1351-1355; Morii, M., and Travis, J. (1985) Biol Chem Hoppe-Seyler 366:19-21: (2) a bovine trypsin inhibitor which inhibits trypsin, chymotrypsin, elastases and plasmin (Hochstrasser, K. and Wachter, E., (1983) Hoppe-Seyler's Z Physiol Chem 364:1679-1687; Hochstrasser, K., et al (1983), Hoppe-Seyler's Z Physiol Chem 364:1689-1696; and (3) a bovine serum basic protease inhibitor (and its precursor) which inhibits trypsin, kallikrein, chymotrypsin, and plasmin (Anderson, S. and Kingston, I.B. (1983) Proc Nat Acad Sci (USA) 80:6838-6842. Based on this level of relatedness to the 168 bp insert protein sequence, one interpretation is that this region of the λAPCP168i4 protein has a function as a protease inhibitor in vivo. While not wishing to be bound by this interpretation, it does suggest that a protease inhibitor based on the sequence of the 168 bp insert protein or a fragment thereof could be useful as a therapeutic reagent for Alzheimer's disease. This or other protease inhibitors, peptidic or non-peptidic, could be used to treat or prevent Alzheimer's disease by a mechanism such as preventing the formation of neuritic plaques. One method of administration might involve nasal delivery of such a peptide (as the blood-brain barrier is known to be more open immediately behind the nasal cavity). Nasal delivery could be accomplished by formulating the protease inhibitor peptide with excipient and an effective amount of an adjuvant, such as the fusidic acid derivatives or a polyoxyethylene ether at a concentration of 0.1-10% (w/w). Stabilizers or disinfectants could optionally be added. The amount of peptide would vary, depending on its efficacy and bioavailability, but could range from 0.1-25% (w/w). Administration would occur by spraying from 10-100 µl of the solution into each side of the nose from 1-4 times a day, although dosing could also be more or less frequent. Other modes of delivery include a solution of inhibitor in a pharmaceutically acceptable excipient where the inhibitor is 0.1-25% (w/w) and where the inhibitor is administered by injection into the bloodstream or into the spinal column, or directly onto the brain. If the inhibitor is non-peptidic, oral dosing may be possible.

### G. Methods and Materials

Most of the techniques which are used to transform cells, construct vectors, extract messenger RNA, prepare cDNA libraries, and the like are widely practiced in the art, and most practitioners are familiar with the standard resource materials which describe specific conditions and procedures. However, for convenience, the following paragraphs may serve as a guideline.

### Hosts and Control Sequences

Both procaryotic and eucaryotic systems may be used to express the β-amyloid core and β-amyloid-related sequences; procaryotic hosts are, of course, the most convenient for cloning procedures. Procaryotes most frequently are represented by various strains of E. coli; however, other microbial strains may also be used. Plasmid vectors which contain replication sites, selectable markers and control sequences derived from a species compatible with the host are used; for example, E. coli is typically transformed using derivatives of pBR322, a plasmid derived from an E. coli species by Bolivar, et al, Gene (1977) 2:95. pBR322 contains genes for ampicillin and tetracycline resistance, and thus provides multiple selectable markers which can be either retained or destroyed in constructing the desired vector. Commonly used procaryotic control sequences which are defined herein to include promoters for transcription initiation, optionally with an operator, along with ribosome binding site sequences, include such commonly used promoters as the beta-lactamase (penicillinase) and lactose (lac) promoter systems (Chang, et al, Nature (1977) 198:1056) and the tryptophan (trp) promoter system (Goeddel, et al Nucleic Acids Res (1980) 8:4057) and the lambda derived P_{L} promoter and N-gene ribosome binding site (Shimatake, et al, Nature (1981) 292:128).

In addition to bacteria, eucaryotic microbes, such as yeast, may also be used as hosts. Laboratory strains of Saccharomyces cerevisiae, Baker's yeast, are most used although a number of other strains or species are commonly available. Vectors employing, for example, the 2 µ origin of replication of Broach, J. R., Meth Enz (1983) 101:307, or other yeast compatible origins of replication (see, for example, Stinchcomb, et al, Nature (1979) 282:39, Tschumper, G., et al, Gene (1980) 10:157 and Clarke, L, et al, Meth Enz (1983) 101:300) may be used. Control sequences for yeast vectors include promoters for the synthesis of glycolytic enzymes (Hess, et al, J Adv Enzyme Reg (1968) 7:149: Holland, et al, Biochemistry (1978) 17:4900). Additional promoters known in the art include the promoter for 3-phosphoglycerate kinase (Hitzeman, et al, J Biol Chem (1980) 255:2073). Other promoters, which have the additional advantage of transcription controlled by growth conditions and/or genetic background are the promoter regions for alcohol dehydrogenase 2, isocytochrome C, acid phosphatase, degradative enzymes associated with nitrogen metabolism, the alpha factor system and enzymes responsible for maltose and galactose utilization. It is also believed terminator sequences are desirable at the 3' end of the coding sequences. Such terminators are found in the 3' untranslated region following the coding sequences in yeast-derived genes.

It is also, of course, possible to express genes encoding polypeptides in eucaryotic host cell cultures derived from multicellular organisms. See, for example, Axel, et al, U.S. Patent No. 4,399,216. These systems have the additional advantage of the ability to splice out introns and thus can be used directly to express genomic fragments. Useful host cell lines include VERO and HeLa cells, and Chinese hamster ovary (CHO) cells. Expression vectors for such cells ordinarily include promoters and control sequences compatible with mammalian cells such as, for example, the commonly used early and late promoters from Simian Virus 40 (SV 40) (Fiers, et al, Nature (1978) 273:113), or other viral promoters such as those derived from polyoma, Adenovirus 2, bovine papilloma virus, or avian sarcoma viruses. The controllable promoter, hMTII (Karin, M., et al, Nature (1982) 299:797-802) may also be used. General aspects of mammalian cell host system transformations have been described by Axel (supra). It now appears, also that "enhancer" regions are important in optimizing expression; these are, generally, sequences found upstream or downstream of the promoter region in noncoding DNA regions. Origins of replication may be obtained, if needed, from viral sources. However, integration into the chromosome is a common mechanism for DNA replication in eucaryotes.

### Transformations

Depending on the host cell used, transformation is done using standard techniques appropriate to such cells. The calcium treatment employing calcium chloride, as described by Cohen, S.N., Proc Natl Acad Sci (USA) (1972) 69:2110, or the RbCl₂ method described in Maniatis, et al, Molecular Cloning: A Laboratory Manual (1982) Cold Spring Harbor Press, p. 254 and Hanahan, D., J Mol Biol (1983) 166:557-580 may be used for procaryotes or other cells which contain substantial cell wall barriers. For mammalian cells without such cell walls, the calcium phosphate precipitation method of Graham and van der Eb, Virology (1978) 52:546, optionally as modified by Wigler, M., et al, Cell (1979) 16:777-785 may be used. Transformations into yeast may be carried out according to the method of Beggs, J.D., Nature (1978) 275:104-109 or of Hinnen, A., et al, Proc Natl Acad Sci (USA) (1978) 75:1929.

### Vector Construction

Construction of suitable vectors containing the desired coding and control sequences employs standard ligation and restriction techniques which are well understood in the art. Isolated plasmids, DNA sequences, or synthesized oligonucleotides are cleaved, tailored, and religated in the form desired.

The DNA sequences which form the vectors are available from a number of sources. Backbone vectors and control systems are generally found on available "host" vectors which are used for the bulk of the sequences in construction. For the pertinent coding sequence, initial construction may be, and usually is, a matter of retrieving the appropriate sequences from cDNA or genomic DNA libraries. However, once the sequence is disclosed it is possible to synthesize the entire gene sequence in vitro starting from the individual nucleoside derivatives. The entire gene sequence for genes of sizeable length, e.g., 500-1000 bp may be prepared by synthesizing individual overlapping complementary oligonucleotides and filling in single stranded nonoverlapping portions using DNA polymerase in the presence of the deoxyribonucleotide triphosphates. This approach has been used successfully in the construction of several genes of known sequence. See, for example, Edge, M. D., Nature (1981) 292:756; Nambair, K. P., et al, Science (1984) 223:1299; Jay, Ernest, J Biol Chem (1984) 259:6311.

Synthetic oligonucleotides are prepared by either the phosphotriester method as described by Edge, et al, Nature (supra) and Duckworth, et al, Nucleic Acids Res (1981) 9:1691 or the phosphoramidite method as described by Beaucage, S.L., and Caruthers, M.H., Tet Letts (1981) 22:1859 and Matteucci, M.D., and Caruthers, M.H., J Am Chem Soc (1981) 103:3185 and can be prepared using commercially available automated oligonucleotide synthesizers. Kinasing of single strands prior to annealing or for labeling is achieved using an excess, e.g., approximately 10 units of polynucleotide kinase to 1 nmole substrate in the presence of 50 mM Tris, pH 7.6, 10 mM MgCl₂, 5 mM dithiothreitol, 1-2 mM ATP, 1.7 pmoles γ32P-ATP (2.9 mCi/mmole), 0.1 mM spermidine, 0.1 mM EDTA.

Once the components of the desired vectors are thus available, they can be excised and ligated using standard restriction and ligation procedures.

Site specific DNA cleavage is performed by treating with the suitable restriction enzyme (or enzymes) under conditions which are generally understood in the art, and the particulars of which are specified by the manufacturer of these commercially available restriction enzymes. See, e.g., New England Biolabs, Product Catalog. In general, about 1 µg of plasmid or DNA sequence is cleaved by one unit of enzyme in about 20 µl of buffer solution; in the examples herein, typically, an excess of restriction enzyme is used to insure complete digestion of the DNA substrate. Incubation times of about one hour to two hours at about 37°C are workable, although variations can be tolerated. After each incubation, protein is removed by extraction with phenol/chloroform, and may be followed by ether extraction, and the nucleic acid recovered from aqueous fractions by precipitation with ethanol. If desired, size separation of the cleaved fragments may be performed by polyacrylamide gel or agarose gel electrophoresis using standard techniques. A general description of size separations is found in Methods in Enzymology (1980) 65:499-560.

Restriction cleaved fragments may be blunt ended by treating with the large fragment of E. coli DNA polymerase I (Klenow) in the presence of the four deoxynucleotide triphosphates (dNTPs) using incubation times of about 15 to 25 min at 20 to 25°C in 50 mM Tris pH 7.6, 50 mM NaCl, 6 mM MgCl₂, 6 mM DTT and 0.1-1.0 mM dNTPs. The Klenow fragment fills in at 5' single-stranded overhangs but chews back protruding 3' single strands, even though the four dNTPs are present. If desired, selective repair can be performed by supplying only one of the, or selected, dNTPs within the limitations dictated by the nature of the overhang. After treatment with Klenow, the mixture is extracted with phenol/chloroform and ethanol precipitated. Treatment under appropriate conditions with S1 nuclease or BAL-31 results in hydrolysis of any single-stranded portion.

Ligations are performed in 15-50 µl volumes under the following standard conditions and temperatures: for example, 20 mM Tris-HCl pH 7.5, 10 mM MgCl₂, 10 mM DTT, 33 µg/ml BSA, 10 mM-50 mM NaCl, and either 40 µM ATP, 0.01-0.02 (Weiss) units T4 DNA ligase at 0°C (for "sticky end" ligation) or 1 mM ATP, 0.3-0.6 (Weiss) units T4 DNA ligase at 14°C (for "blunt end" ligation). Intermolecular "sticky end" ligations are usually performed at 33-100 µg/ml total DNA concentrations (5-100 nM total end concentration). Intermolecular blunt end ligations are performed at 1 µM total ends concentration.

In vector construction employing "vector fragments", the vector fragment is commonly treated with bacterial alkaline phosphatase (BAP) or calf intestinal alkaline phosphatase (CIP) in order to remove the 5' phosphate and prevent self-ligation of the vector. Digestions are conducted at pH 8 in approximately 10 mM Tris-HCl, 1 mM EDTA using about 1 unit of BAP or CIP per µg of vector at 60° for about one hour. In order to recover the nucleic acid fragments, the preparation is extracted with phenol/chloroform and ethanol precipitated. Alternatively, religation can be prevented in vectors which have been double digested by additional restriction enzyme digestion and separation of the unwanted fragments.

For portions of vectors derived from cDNA or genomic DNA which require sequence modifications, site specific primer directed mutagenesis may be used (Zoller, M.J., and Smith, M. Nucleic Acids Res (1982) 10:6487-6500 and Adelman, J.P., et al, DNA (1983) 2:183-193). This is conducted using a primer synthetic oligonucleotide complementary to a single stranded phage DNA to be mutagenized except for limited mismatching, representing the desired mutation. Briefly, the synthetic oligonucleotide is used as a primer to direct synthesis of a strand complementary to the phage, and the resulting partially or fully double-stranded DNA is transformed into a phage-supporting host bacterium. Cultures of the transformed bacteria are plated in top agar, permitting plaque formation from single cells which harbor the phage.

Theoretically, 50% of the new plaques will contain the phage having, as a single strand, the mutated form; 50% will have the original sequence. The resulting plaques are washed after hybridization with kinased synthetic primer at a wash temperature which permits binding of an exact match, but at which the mismatches with the original strand are sufficient to prevent binding. Plaques which hybridize with the probe are then picked, cultured, and the DNA recovered.

### Verification of Construction

In the constructions set forth below, correct ligations for plasmid construction are confirmed by first transforming E. coli strain MC1061 obtained from Dr. M. Casadaban (Casadaban, M., et al, J Mol Biol (1980) 138:179-207) or other suitable host with the ligation mixture. Successful transformants are selected by ampicillin, tetracycline or other antibiotic resistance or using other markers depending on the mode of plasmid construction, as is understood in the art. Plasmids from the transformants are then prepared according to the method of Clewell, D.B., et al, Proc Natl Acad Sci (USA) (1969) 62:1159, optionally following chloramphenicol amplification (Clewell, D.B., J Bacteriol (1972) 110:667). Several mini DNA preps are commonly used, e.g., Holmes, D.S., et al, Anal Biochem (1981) 114:193-197 and Birnboim, H.C., et al, Nucleic Acids Res (1979) 7:1513-1523. The isolated DNA is analyzed by restriction and/or sequenced by the dideoxy nucleotide method of Sanger, F., et al, Proc Natl Acad Sci (USA) (1977) 74:5463 as further described by Messing, et al, Nucleic Acids Res (1981) 9:309, or by the method of Maxam, et al, Methods in Enzymology (1980) 65:499.

The invention will be further described by the following examples. These are provided only to illustrate embodiments of the invention and are not to be construed as limitations on the invention's scope.

### Example 1

### Isolation of a Genomic Clone and cDNA Clones Encoding β-amyloid Core Protein and β-amyloid-related Proteins

A human genomic library in Charon 4A λ-phage was screened using a six-fold degenerate 38 mer probe corresponding to the first 13 amino acids of the 28 amino acid sequence N-terminal sequence. This probe, 
3'CTGCGACTTAAGGCCGTGCTGAGICCGATGCTTCAGGT-5'
G T
T
 wherein I is inosine, when used to screen the human genomic library yielded a strongly hybridizing colony designated λSM2. λSM2 DNA was isolated and partially sequenced with the results shown in Figure 2. The sequenced portion is only a small fraction of the approximately 10-20 kb insert in the phage isolated from the genomic library.

A probe was constructed from the HindIII/RsaI fragment representing approximately positions 201-294. The genomic probe was used to screen a cDNA library prepared in λgt10 using standard techniques from brain tissue of a 55 year old man with no evidence of Alzheimer's disease. The three clones designated λSM2W4, λSM2W3 and λSM2W9 were identified.

### Example 2

The genomic and cDNA sequences described above can be used to prepare recombinant protein in an efficient expression system. Genomic DNA can be utilized in cells, such as mammalian cells, capable of processing introns. Bacterial cells can be utilized for expression of cDNA sequences.

### Bacterial Expression of β-Amyloid-Related Protein (655-751) and Production of Antisera

### A. Construction of plasmid pAPCP118-3.

Construction of an E. coli expression vector for human β-amyloid-related protein (655-751) required the joining of three DNA fragments: (1) a plasmid backbone (consisting of replication functions, ampicillin resistance gene, tryptophan promoter/operator, ribosome binding site, DNA encoding the amino terminus of E. coli beta-galactosidase (7 amino acids) followed by six threonine residues, and transcription termination signals), (2) a fragment of the β-amyloid-related DNA encoding amino acids 655-728, of Figure 1 and (3) a synthetic fragment of the β-amyloid-related DNA encoding amino acids 729-751 of Figure 1 and the stop codon UAA.

The plasmid backbone referred to above is derived from pTRP83-1. Plasmid pTRP83-1 is a bacterial expression plasmid which was constructed in the following manner:

### 1. Construction of the Synthetic Tryptophan Operon Promoter and Operator Regulatory Sequence

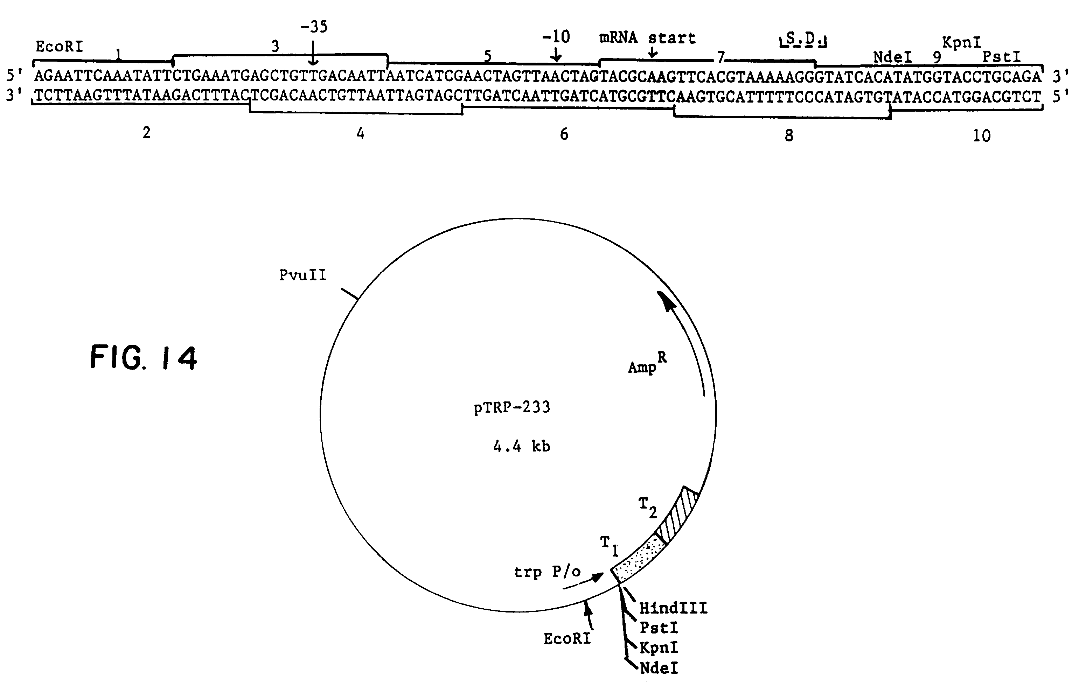

The ten oligodeoxynucleotides shown in Figure 14 were synthesized by the phosphotriester method and purified. 500 pmole of each oligodeoxynucleotide except 1 and 10 were phosphorylated individually in 20 µl containing 60 mM Tris-HCl, pH 8, 15 mM DTT, 10 mM MgCl₂, 20 µCi of [γ-³²P]-ATP and 20 units of polynucleotide kinase (P/L Biochemicals) for 30 min. at 37°C. This was followed by the addition of 10 µl containing 60 mM Tris-HCl, pH 8, 15 mM DTT, 10 mM MgCl₂, 1.5 mM ATP and 20 additional units of polynucleotide kinase followed by another 30 min incubation at 37°C. Following incubation the samples were incubated at 100°C for 5 min. 500 pmole of oligodeoxynucleotides 1 and 10 were diluted to 30µl in the above buffer without ATP.

16.7 pmole of each oligodeoxynucleotide constituting a double stranded pair (e.g. oligodeoxynucleotides 1 and 2, 3 and 4 etc. Figure 14 were mixed and incubated at 90°C for 2 min followed by slow cooling to room temperature. Each pair was then combined with the others in the construction and extracted with phenol/chloroform followed by ethanol precipitation. The oligodeoxynucleotide pairs were reconstituted in 30 µl containing 5 mM Tris-HCl, pH 8, 10 mM MgCl₂, 20 mM DTT, heated to 50°C for 10 min and allowed to cool to room temperature followed by the addition of ATP to a final concentration of 0.5 mM. 800 units of T4 DNA ligase were then added and the mixture incubated at 12.5°C for 12-16 hours.

The ligation mixture was extracted with phenol/chloroform and the DNA ethanol precipitated. The dried DNA was reconstituted in 30 µl and digested with EcoRI and PstI for 1 hour at 37°C. The mixture was extracted with phenol/chloroform and ethanol precipitated followed by separation of the various double stranded DNA segments by electrophoresis on an 8% polyacrylamide gel, according to the method of Laemmli et al, Nature (1970) 227:680. The DNA fragments were visualized by wet gel autoradiography and a band corresponding to approximately 100 bp in length was cut out and eluted overnight as described. The excised synthetic DNA fragment was ligated to plasmids M13-mp8 or M13-mp9 (Messing and Vieira, (1982) Gene 19:259-268) similarly digested with EcoRI and PstI, and submitted to dideoxynucleotide sequence analysis to confirm the designed sequence. This designed sequence contains the promoter (-35 and -10 regions) and operator regions of the tryptophan operon (trp) as well as the ribosome binding region of the tryptophan operon leader peptide. Analogous sequences to that shown in Figure 14 have been proven to be useful in the expression of heterologous proteins in E. coli (Hallewell, R.A., and Emtage, S., (1980) Gene 9:27-47, Ikehara, M., et al, Proc Natl Acad Sci (USA) (1984) 81:5956-5960).

### 2. Construction of the Synthetic trp Promoter/Operator Containing Plasmid pTRP233

Plasmid pKK233-2 (Amann, E. and Brosius, J. (1985) Gene 40:183 was digested to completion with NdeI and the ends were made blunt with 5 units of E. coli polymerase I, Klenow fragment (Boehringer-Mannheim, Inc.) and the addition of all four dNTPs to 50µM. This was incubated at 25°C for 20 min. Following phenol/chloroform extraction and ethanol precipitation, the NdeI-digested DNA was ligated and transformed into E. coli (Nakamura, K. et al (1982) J Mol Appl Genet 1:289-299). The resulting plasmid lacking the NdeI site was designated pKK-233-2-Nde.

Twenty nanograms of plasmid pKK-233-2-Nde was digested to completion with EcoRI and PstI followed by calf intestinal phosphatase treatment. Fifty nanograms of the synthetic trp promoter/operator sequence obtained from M13 RF, by digesting with EcoRI and PstI, were mixed with ten nanograms of EcoRI and PstI-digested pKK-233-2-Nde and ligated with T4-DNA ligase, followed by transformation into E. coli JA221 1pp⁻/I'lacI. Transformants were screened for the presence of plasmid DNA containing the 100 bp EcoRI-PstI synthetic trp promoter/operator; the correct plasmid was then isolated and designated pTRP233.

pTRP233 was digested with EcoRI, the ends blunted with Klenow, and ligated to remove the EcoRI restriction site. The plasmid was next digested with NdeI and HindIII and an NdeI-EcoRI-HindIII fragment encoding beta-gal-(thr)6 between the NdeI and EcoRI sites was inserted to create plasmid pTRP83-1.

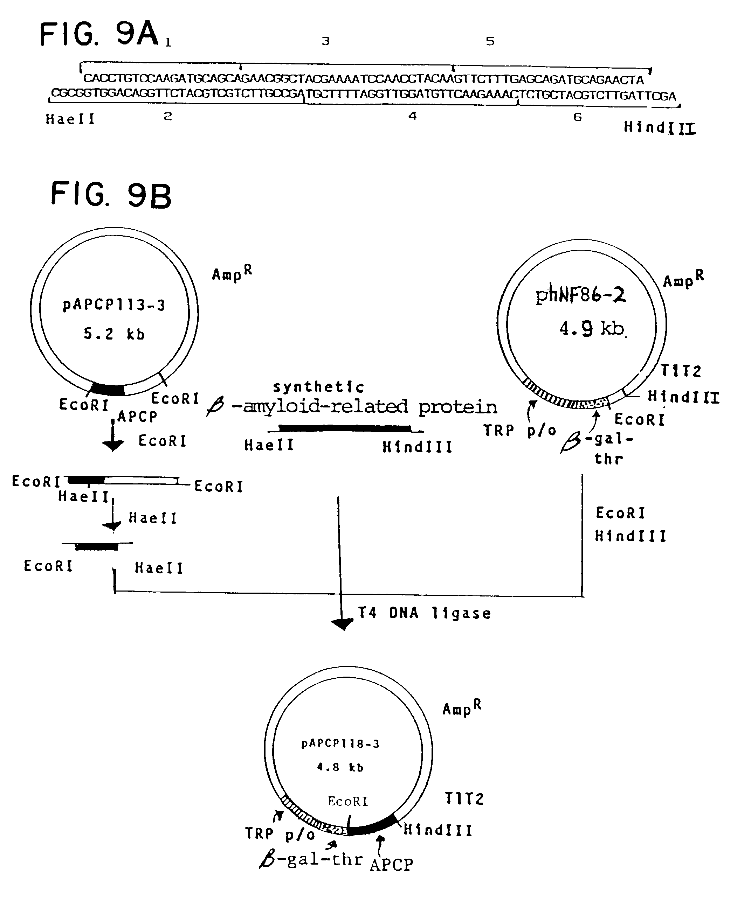

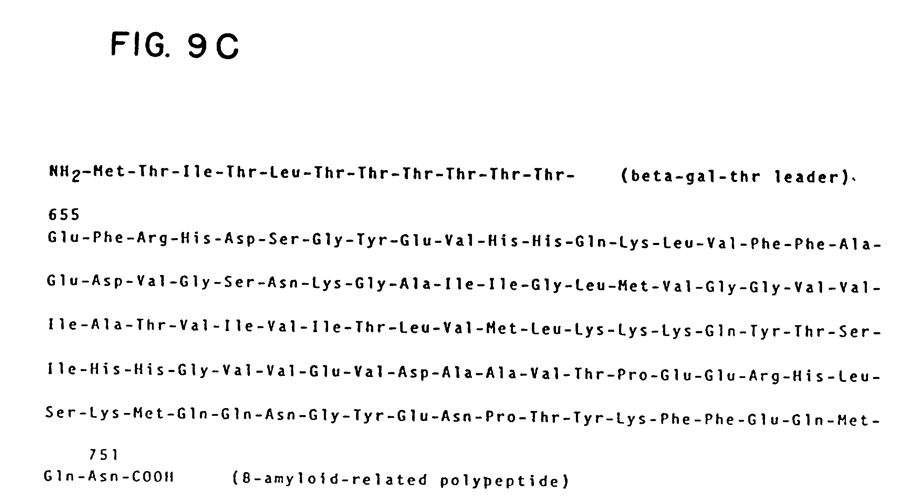

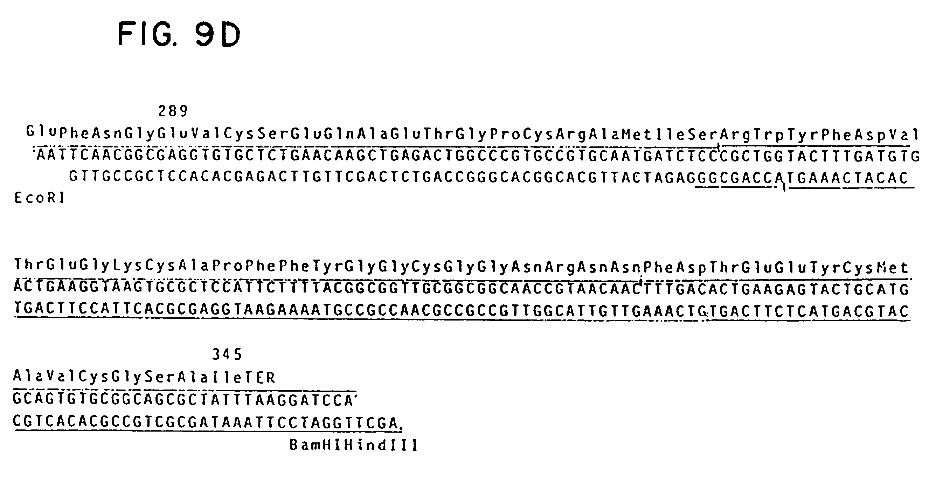

Plasmid pTRP83-1 was then digested with EcoRI and HindIII restriction endonucleases and the digest was electrophoresed in a 0.6% agarose gel (Maniatis, T. et al at pp. 157-160). The large fragment containing the plasmid backbone was eluted from the gel. Next, the EcoRI fragment from plasmid pAPCP113-3 containing β-amyloid-related sequences derived from λSM2W3 (corresponding to amino acids 655-751 of Figure 1 and 500 bp of 3'-untranslated sequences) was digested with HaeII restriction endonuclease and electrophoresed in a 12% polyacrylamide gel. The approximately 230 bp EcoRI-HaeII fragment (containing β-amyloid-related sequences encoding amino acids 655-728 was eluted. The remaining portion of the β-amyloid-related sequences of Figure 1 encoding amino acids from 728-751 were prepared using the six oligodeoxynucleotides illustrated in Figure 9. 500 pmole of each oligodeoxynucleotide except for 1 and 6 were phosphorylated individually. 167 pmole of each oligodeoxynucleotide constituting a pair (e.g. 1 and 2, 2 and 3, etc.) were mixed and incubated at 90°C for 2 min followed by slow cooling to room temperature. Each pair was then combined with the others and extracted with phenol/chloroform followed by ethanol precipitation. The pairs were reconstituted in 30 µl containing 5 mM Tris-HCl, pH 8, 10 mM MgCl₂, 20 mM DTT, heated to 50°C for 10 min, and allowed to cool to room temperature. ATP was added to a final concentration of 0.5 mM, 800 units of T4 DNA ligase was added and the mixture incubated at 12° C for 12-16 hr. The ligation was electrophoresed in a 12% polyacrylamide gel and the 79 bp HaeII-HindIII synthetic fragment was eluted.

The EcoRI-HindIII plasmid backbone of pTRP83-1. the approximately 230 bp EcoRI-HaeII β-amyloid cDNA fragment, and the 79 bp synthetic HaeII-HindIII β-amyloid fragment were ligated at 12°C for 12-16 hr. E. coli strain MC1061 was transformed with the ligation mixture (Maniatis, T. et al, pp. 250-251) and the resulting ampicillin resistant colonies were grown overnight in 1 ml of L broth supplemented with 100 µg/ml ampicillin sulfate. Plasmid DNA was prepared by the alkaline lysis method (Maniatis et al, pp. 368-369). Plasmids were screened for the correct inserts by digestion with EcoRI and HindIII. A plasmid releasing an approximately 300 bp EcoRI-HindIII fragment was designated pAPCP118-3.

### B. Expression of β-amyloid-related Fusion Polypeptide (655-751).

The plasmid pAPCP118-3 expresses a 110 amino acid beta-galactosidase-threonine-β-amyloid-related fusion protein under the control of the E. coli tryptophan promoter/operator. E. coli strain W3110 was transformed with plasmid pAPCP118-3 and one of the resulting ampicillin resistant colonies was grown for 12-16 hr at 37°C in media containing M9 minimal salts (Miller, J., Experiments in Molecular Genetics, Cold Spring Harbor Laboratory, Cold Spring Harbor, New York) supplemented with glucose (0.4%), thiamine (2µg/ml), MgS04∗7H20 (200 µg/ml), tryptophan (40 µg/ml), casamino acids (0.5%), and ampicillin (100 µg/ml). Expression was induced by dilution of the culture 100-fold into new media with reduced tryptophan (4 µg/ml) for 2 hr followed by the addition of 3-beta-indoleacrylic acid at a final concentration of 25 µg/ml. Expression of beta-gal-thr-β-amyloid (655-751) fusion protein occurs at the level of 10-20% of total cell protein, and is present in the form of inclusion bodies which can be visualized by phase contrast microscopy (1000 × magnification). The cells were harvested 6 hr after the addition of the 3-beta-indoleacrylic acid by centrifugation, washed with 10 mM Tris-HCl, pH 7.5, and the cell pellet frozen at -20°C.

### C. Purification of Beta-gal-thr-β-amyloid (655-751) Fusion Protein for Preparation of Antiserum.

A cell pellet from 500 ml of culture was resuspended in 40 ml of 10 mM Tris-HCl, pH 7.5, 0.6 M NaCl, and incubated with 8 mg of lysozyme and the protease inhibitors phenylmethylsulfonylfluoride (PMSF) and aprotinin (0.5 mM and 25 µg/ml respectively) for 10 min at 4°C. Solutions of the two detergents, sodium deoxycholate (480 µl of 10% solution) and NP-40 (240 µl of 20% solution), were then added for an additional 10 min incubation at 4°C. The cell pellet was sonicated to disrupt cells and free inclusion bodies. RNAse (10 µg/ml) and DNAse (10 µg/ml) were added and the mixture stirred for 30 min at room temperature to digest RNA and DNA. The inclusion bodies (and some cell debris) were collected by centrifugation for 10 min at 5000 rpm (SA600 rotor). The supernatant was discarded and the pellet boiled in protein gel sample buffer for 20 min to solubilize the fusion protein. The fusion protein was then purified by electrophoresis in 12% SDS/polyacrylamide gels (Laemmli, U.-K., Nature (1970), 227:680). The edges of each gel were removed and stained with Coomassie blue to visualize the 15 kilodalton (kD) fusion protein. They were then realigned with the gel so that the region of the gel containing the fusion protein could be excised. The polyacrylamide was then crushed through a series of needles (16 gauge down to 22 gauge) with the addition of physiological saline to keep the polyacrylamide moist. The polyacrylamide/fusion protein crush was mixed with adjuvant [RIBI(RAS)] just prior to immunization of the rabbits. Approximately 150-200 µg of fusion protein was administered per animal for the first immunization. Subsequent immunizations use 50-100 µg of fusion protein.

### D. Western Blot Analysis of β-amyloid Synpep Antisera Using Beta-gal-thr-β-amyloid (655-751) Fusion Protein.

Cell pellets of E. coli W3110 (pAPCP118-3) and W3110 (pTRP83-1) cultures induced with 3-beta-indoleacrylic acid were boiled in Laemmli gel sample buffer and electrophoresed in 12% SDS polyacrylamide. The second transformed strain is a negative control which contains all proteins except for the beta-gal-thr-β-amyloid (655-741) fusion. The gels were then electroblotted to nitrocellulose, incubated first with APCP synpep antisera collected from immunized rabbits, and then incubated with ¹²⁵I-Staphylococcus protein A to identify bound antibody (Johnson, D.A. et al, Gene Anal Tech (1984) 1:3). An autoradiogram was generated from these nitrocellulose filters which demonstrated crossreactivity between anti-APCP3 serum and the fusion protein, Synpep APCP3 is comprised of amino acids 705-719 of Figure 1 which are included within the β-amyloid portion of the fusion protein. Cross-reactivity was also observed for other β-amyloid synpep antisera.

### Example 3

### Generation of Polyclonal and Monoclonal Antibodies Against β-amyloid-related Protein Using Live Recombinant Vaccinia Virus

### 1. Construction of Plasmid pFL4T4B.

The construction of the plasmid which allowed for the generation of polyclonal and monoclonal antibodies is schematically represented in Figure 10. Plasmid pGEM-3™ (Promega-Biotec) was EcoRI-digested and treated with calf intestinal phosphatase in accordance with Maniatis, et al. Fifty nanograms of the purified 1.06Kb EcoRI fragment derived from λAPCP168i4 were mixed with 10 nanograms EcoRI digested pGEM-3™ and incubated with T4 DNA ligase in a total volume of 20 ul for 30 min at 25°C. E. coli strain MC1061 was made competent for transformation by the CaCl₂ method and transformed with the ligation mix. Resulting ampicillin resistant colonies were grown overnight in 2 ml L-amp broth from which plasmid DNA was prepared by the Triton-lysis method (Maniatis et al). Plasmids were screened for the correct orientation by digestion with HindIII. A plasmid having 150 and 3700 bp HindIII restriction fragments was chosen and designated p4BI. The resulting plasmid p4BI was digested with HindIII, religated with T4 ligase for 30 minutes at 25°C and competent MC1061 cells were transformed with the ligation mixture. Plasmids were screened for loss of the 130 bp HindIII fragment by EcoRI digestion. A plasmid containing a single EcoRI site was chosen and designated p4BΔRI. Ten nanograms of plasmid p4BΔRI was EcoRI-digested, treated with calf intestinal alkaline phosphatase, and ligated with 100 nanograms of the purified ∼2 kb EcoRI fragment derived from λAPCP168i4. The ligation mixture was used to transform competent MC1061 cells. Resulting ampicillin-resistant colonies were grown overnight in L-amp broth and plasmid DNA was prepared. Plasmids were screened for the correct orientation by digestion with BamHI and HindIII. A plasmid having a 1.5 kb BamHI and an ∼ 1.5 kb BamHI-HindIII fragment was chosen and designated p4T4B. Plasmid p4T4B was digested with SmaI and XmnI and the resulting ∼ 2.7kb fragment was eluted from 0.8% agarose followed by ethanol precipitation, dryed in vacuo and resuspended in dH₂O.

Five µg of the vaccinia virus expression vector pSC11 (Chakrabarti et al (1985) Mol Cell Biol 5:3403-3409) were digested to completion with SmaI followed by treatment with calf intestinal phosphotase. Five hundred nanograms of the purified ∼ 2.7 kb SmaI-XmnI fragment derived from p4T4B were mixed with fifty nanograms of SmaI digested PSC11 and incubated with T4 DNA ligase in a total volume of 20 µl for 16 hours at 15°C overnight. E. coli strain MC1061 was transformed with the ligation mix. Resulting ampicillin resistant colonies were grown overnight and plasmid DNA was isolated by the rapid boiling method (Maniatis et al). Plasmids were screened for insertion and correct orientation by digestion with EcoRl. A plasmid having both an ∼2500 bp and an ∼600 bp EcoRl fragment was chosen and designated pFL4T4BV.

Monoclonal and polyclonal antibodies against full length β-amyloid-related protein is generated by using a novel method described by Yilma, T., et al (Hybridoma (1987) 6:329-337). Briefly, the method enables the production of antibodies to a specified protein without the need for a purified antigen (protein) in either the immunization or screening phase of the procedure. The methods make use of the vaccinia virus cloning vectors (Smith et al, Nature (1983) 302:490-495) which can be genetically engineered to carry isolated genes. The infectious recombinant vaccinia virus may then be used to immunize mice. Two weeks after infection, mice are sacrificed and their spleen cells are fused with myeloma cells for monoclonal antibody production as described in the classical approach developed by Kohler and Milstein (1973) Nature 256:495. Alternatively, rabbits can be conventionally immunized with the infectious vaccinia virus recombinant to generate polyclonal antisera.

Ten µg of plasmid p4T4BV is used to transfect CV-1 monkey kidney cells infected with wild-type vaccinia virus according to standard methods (Mackett et al, J Virol (1984) 49:857-864). TK⁻ recombinants are isolated by plaque assay on TK⁻ cells in the presence of 25 µg/ml Bromodeoxyuridine (BUdR). For plaque assays involving blue color production, as in the case of the PSC11 vaccinia virus coexpression vector, 300 µg of X-Gal per milliliter is placed in the agarose overlay, and plaques visualized after 4-6 hrs at 37°C. Plaques are purified two to three times in succession. DNA from the recombinant virus is examined by restriction endonuclease analysis and DNA hybridization to ³²P-nick-translated 2091 bp EcoRI fragment from λAPCP168i4 to confirm the predicted structure.

Recombinant virus carrying the complete β-amyloid-related cDNA sequence of λAPCP168i4 is isolated and amplified to high titre (1×10⁸⁻⁹ pFu/ml). These recombinant viruses are used to immunize rabbits and mice for the subsequent production of polyclonal and monoclonal antibodies respectively, against full length β-amyloid-related protein(s) using well established methods. The various antisera are screened either for their ability to specifically immunoprecipitate the correct size protein from ³⁵S-methionine-labeled CV-1 cells which have been infected with an β-amyloid-related protein virus recombinant or for their ability to detect denatured protein on a western blot of similar cells which have not been exposed to radiolabeled amino acid.

### Example 4

### Expression of β-amyloid-Related Protein (1-751) in Cultured Mammalian Cells.

To facilitate the expression of β-amyloid-related protein in mammalian cells, a plasmid is constructed such that the coding segment for the protein is fused to a powerful regulated promoter derived from the human metallothionen II (hMTII) gene. This procedure is performed in two steps. First an expression vector pMTSV40 polyA Bam was derived from phGH-SV(10) vector by digestion of phGH-SV(10) with BamHI and SmaI restriction enzymes, followed by incubation with DNA polymerase I (Klenow fragment) in order to create blunt-ended molecules. The blunt ends are subsequently ligated to BamHI linkers, cut with BamHI, and religated to allow for recircularization. This step removes all of the human growth hormone genomic sequence from phGH-SV(10) except for most of the 3' untranslated region of the mRNA and genomic sequences encoding putative 3' transcriptional stop and processing signals. For the mammalian cell expression construct, pMTSV40 polyA Bam is BamHI-digested, then incubated with all four nucleoside triphosphates and with DNA polymerase I to create blunt ends. This fragment is subsequently ligated with the purified 2678 bp SmaI-XmnI fragment derived from p4T4B (described previously). The recombinant molecules are introduced into MC1061 by transformation.

Chinese hamster ovary (CHO)-K1 cells are grown in a medium composed of a 1:1 mixture of F12 medium and DME medium with 10% fetal calf serum. The competent cells are co-transformed with the recombinant expression vector and pSU2:NEO (Southern, P., et al, (1982) J Mol Appl Genet 1:327-341). pSV2:NEO contains a functional gene conferring resistance to the neomycin analog G418. In the transformation, 500 ng of pSV2:NEO and 5 µg of the recombinant vector are applied to a 60 mm dish of CHO cells as a calcium phosphate-DNA co-precipitate as described by Graham, F.L. and Van der Eb, A.J. (1973) Virology 52:456-467. Growth of the cells in the antibiotic G418 as described by Southern et al will yield a pool of stably transfected CHO cells containing expression vector DNA with the capacity to express β-amyloid-related mRNA and protein.

### Example 5

### Expression of β-amyloid-related Protein (652-751) in Cultured Mammalian Cells.

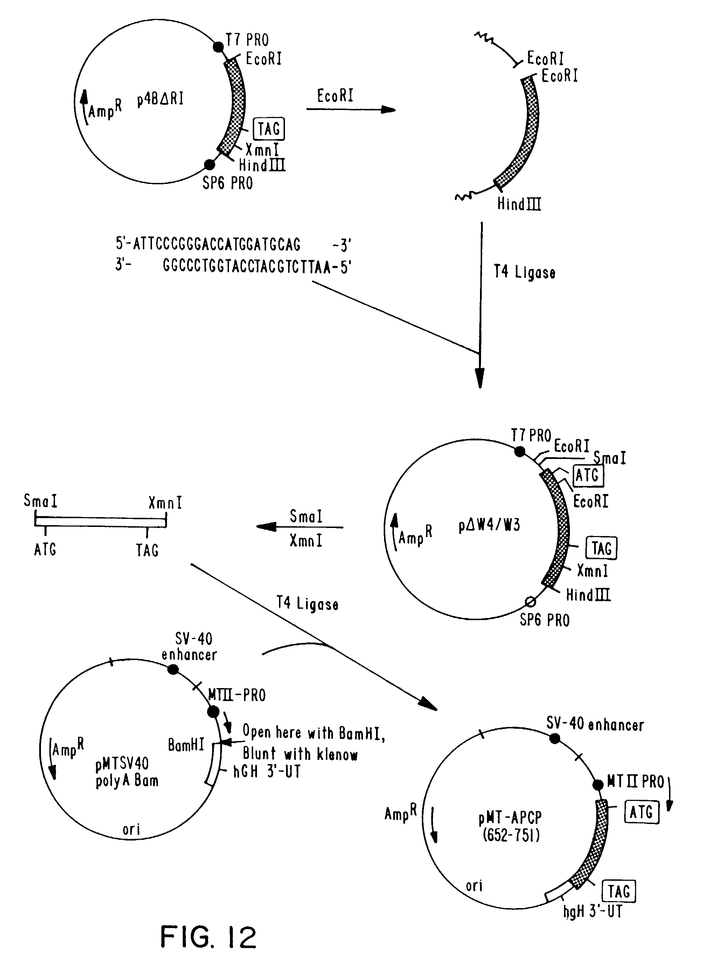

A mammalian cell expression vector encoding for the production of a β-amyloid-related protein can be constructed as shown in Figure 12 as follows: the p4BΔRI vector of Figure 10 is linearized by digestion with EcoRI . The vector is mixed with two oligonucleotides having the sequences: 
5'-ATTCCCGGGACCATGGATGCAG-3'
3'-GGCCCTGGTACCTACGTCTTAA-5'
 and ligated using T4 DNA ligase. These oligonucleotides reconstruct the Met-Asp-Ala codons of λSM2W4 and preceed them by EcoRI and SmaI sites and follow them with another EcoRI site.

Competant E. coli strain DH1 cells are transformed with the mixture and ampicillin-resistant bacteria are selected by growth on L-Amp plates. A transformant containing the oligonucleotide pair inserted into the EcoRI site in the proper orientation is selected by standard screening techniques and designated PΔW4/W3. Plasmid DNA PΔW4/W3 is digested with SmaI and XmnI to remove sequences encoding the β-amyloid-related protein described in Figure 5 and the correct piece is isolated by gel purification.

This piece can then be inserted into the mammalian cell expression vector pMTSV40 polyA Bam which has been linearized with BamHI and rendered blunt-ended as described above in Example 4. The resulting vector, pMT-APCP (652-751) can be used for the production of the β-amyloid-related protein (652-751).

### Example 6

### Assay to Distinguish Genetic Variants of β-Amyloid-Related Protein mRNA Species

The ability to distinguish between genetic variants of β-amyloid-related protein mRNA species using oligonucleotide probes is demonstrated herein.

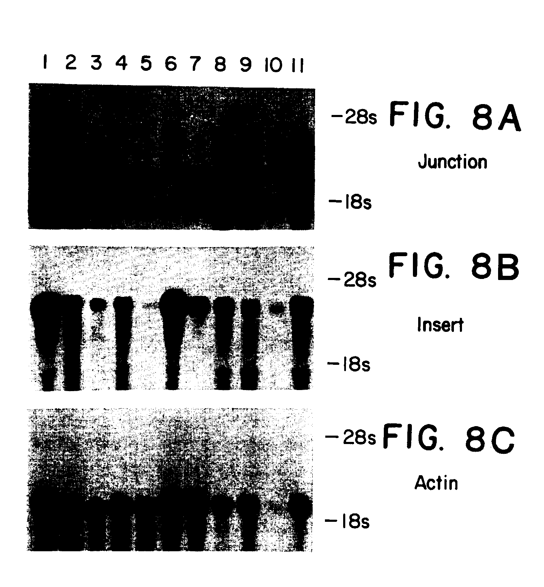

A diagnostic assay for Alzheimer's disease might take the form of distinguishing between two closely related genetic variants of β-amyloid-related proteins or their mRNAs, and quantitating the relative levels of expression of these proteins or mRNAs. Figure 8 provides an example of the use of the invention sequences to provide a standard for the diagnostic assay.

Total cellular RNA or cytoplasmic RNA was prepared from human cells in culture or human brain tissue (Alzheimer's brain or normal brain) with or without removal of nuclei (cytoplasmic or total, respectively) by the guanidine thiocyanate/CsCl method as described by Maniatis et al. The samples corresponding to the numbering in Figure 8 are: (1) total RNA from IMR-32 cells (ATCC #CCL127), a mixed neuroblastoma and fibroblast culture; (2) total RNA from MRC5 cells (ATCC #CCL171), a normal fibroblast; (3) total RNA from HeLa cells (ATCC #CCL2.2), an epitheloid cell; (4) cytoplasmic RNA from MRC5 cells; (5) cytoplasmic RNA from HeLa cells; (6) total RNA from HL-60 cells (ATCC #CCL240), a promyelocytic leukemia; (7) total RNA from HL-60 cells which have been treated with 12-tetra-decanoyl-phorbol-13-acetate to induce differentiation of the cells to macrophages; (8) total RNA from normal cerebellum samples; (9) total RNA from normal frontal cortex samples; (10) total RNA from an Alzheimer's individual's frontal cortex; and (11) total RNA from a normal parietal cortex. RNA was fractionated by oligo-dT cellulose chromatography, electrophoresed on a formaldehyde agarose gel, and blot-transferred to nitrocellulose (all as described in Maniatis et al). Filters were baked, prehybridized and hybridized to the indicated probes according to standard protocols.

The probes indicated are: (1) Junction, a 30 base oligonucleotide #2733, specific for the Kang et al sequence, as described above in the detailed description of the invention; (2) Insert, a 60 base oligonucleotide #2734 specific for the β-amyloid-related sequences described in Figure 1, and as described above; and (3) an 1800 bp human actin cDNA insert, isolated from the plasmid pHFBA-1 (Ponte, P., et al (1984) Nuc Acids Res 12:1687-1696. Oligonucleotide probes were end-labeled with [³²P]-dCTP by incubation with terminal transferase according to manufacturer's suggestions. Actin insert was radiolabeled with [³²P]-CTP by nick-translation. After hybridization, the filters hybridized to oligonucleotides were washed at 1 × S.S.C., 55° C. The filter hybridized to actin was washed at 0.1 × SSC at 55°C. Filters were then exposed to X-ray film to produce the autoradiogram shown. The insert probe detects the β-amyloid related protein mRNA described in Figure 1 in all samples examined. The junction probe detects the β-amyloid-related mRNA described by Kang et al in all cells except HeLa and MRC5. The actin probe is a control which is expected to hybridize to an abundant RNA in all cells.

### Example 7

### Bacterial Expression of β-Amyloid-Related Protein (289-345)

### A. Construction of Plasmid pAPCP125-2.

A synthetic gene was assembled according to the teaching of Example 2 for β-amyloid-related protein (289-345) from three pairs of oligodeoxyribonucleotides (illustrated in Figure 9D) utilizing E. coli preferred codon choice for highly expressed genes, and a hydroxylamine cleavage site (Asn-Gly) was inserted preceding amino acid 289 (Glu) to permit release of the polypeptide from a fusion protein. The expression vector pTRP83-1 was digested with restriction endonucleases EcoRI and HindIII and the linearized plasmid purified from a 0.6% agarose gel. Fifty µg of plasmid DNA and 200 µg of synthetic gene DNA were ligated using T4 DNA ligase and E. coli MC1061 was transformed with the ligation. Ampicillin-resistant colonies were grown overnight in L broth containing 100 µg/ml ampicillin and alkaline plasmid preps were made. The resulting plasmid DNA was digested with BamHI restriction endonuclease to confirm insertion of the gene within the vector by release of an approximately 350 bp fragment. One plasmid receiving the synthetic gene insert was designated pAPCP125-2.

### B. Expression of β-Amyloid-Related Fusion Polypeptide (289-345).

The plasmid pAPCP125-2 is designed to express a 74 amino acid beta-galactosidase-threonine-β-amyloid-related fusion protein under the control of the E. coli tryptophan promoter/operator. E. coli strain W3110 is transformed with plasmid pAPCP125-2 and one of the resulting ampicillin resistant colonies is grown as described in Example 2. Expression is induced by the addition of 3-beta-indoleacrylic acid at a final concentration of 25 µg/ml. After 5 hrs induction, a 1 ml aliquot of cells is withdrawn from the culture, harvested by centrifugation, then boiled in 100 µl of Laemmli protein sample buffer for electrophoresis through a 16% SDS-polyacrylamide gel by standard methodologies. Assessment of inclusion body formation is made by phase contrast microscopy (1000X). Expression levels are estimated by Coomassie blue staining of the gel followed by densitometer scan to quantitate the intensity of protein bands. Cells to be used for protein purification are harvested by centrifugation, washed with 10 mM Tris-HCl, pH 7.5, and the cell pellet frozen at -20°C until needed.

### C. Purification of Beta-gal-thr-β-amyloid-related Protein (289-345).

The fusion protein is purified as described for the beta-gal-thr-β-amyloid-related (655-751) fusion protein (Example 2) in the absence of PMSF and aprotinin. A series of washes from 2 M urea to 4 M urea removes other proteins and further enriches fusion protein found in inclusion bodies. If further purification is desired, the fusion protein is solubilized in 6-8 M urea, and a gel filtration or ion exchange chromatography step is included. If not, the fusion protein is solubilized in 6 M guanidium hydrochloride with hydroxylamine under the conditions described by Moks et al, Biochem (1987) 26:5239-5244 for cleavage between the Asn and Gly residues releasing β-amyloid-related protein (289-345) with a Gly residue at its amino-terminus. The cleaved peptides are purified by reversed phase high pressure liquid chromatography, ion exchange or gel filtration chromatography. The purified β-amyloid-related protein is then reduced and reoxidized by methods described by Tan and Kaiser, J Org Chem (1976) 41:2787 and Biochemistry (1977) 16:1531-1541, to reform disulfide bonds between the six Cys residues. Successful reoxidation of bovine pancreatic trypsin inhibitor (aprotinin) also containing six Cys residues and produced in E. coli has been accomplished by these methods (von Wilcken-Bergmann et al, EMBO Journal (1986) 5:3219-3225.

While preferred embodiments of making and using the invention have been described, it will be appreciated that various changes and modifications can be made without departing from the invention.

The following cultures have been deposited with the American Type Culture Collection (ATCC), Rockville, MD, USA for patent purposes. Bacteriophage phages λSM2, λSM2W9, and λAPCP168i4 were deposited under the conditions specified by the Budapest Treaty on the International Recognition of the Deposit of Microorganisms (Budapest Treaty).

| Culture | Accession No. | Deposit Date |
|---|---|---|
| λSM2 | 40279 | 13 November 1986 |
| SM2W4 | 40299 | 29 December 1986 |
| SM2W3 | 40300 | 29 December 1986 |
| λSM2W9 | 40304 | 29 January 1987 |
| λAPCP168i4 | 40347 | 1 July 1987 |

Availability of the deposited strains are not to be construed as a license to practice the invention in contravention of the rights granted under the authority of any government in accordance with its patent laws.

## Claims (Claims for the following Contracting State(s): AT, BE, CH, LI, DE, FR, GB, IT, LU, NL, SE)

1. A DNA sequence useful in the prognosis and diagnosis of Alzheimer's disease in human subjects comprising the DNA sequence of Figure 1 or a subfragment thereof provided that such a subfragment does not consist of DNA encoding the 28 amino-terminal amino acid residues of the β-amyloid core protein or a subfragment thereof and such a subfragment contains the sequence encoding amino acids numbers 289-345.

2. DNA according to claim 1 which comprises the 168 base pair insert fragment of the β-amyloid-related gene product of bacteriophage λAPCP168i4, deposited as ATCC 40347.

3. DNA according to claim 1 or 2 wherein the subfragment corresponds to a naturally occurring restriction fragment.

4. DNA according to claim 1 which encodes a subfragment having the amino acid sequence:

Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile

5. DNA according to claim 4, having the sequence of the 168 base pair insert fragment of the β-amyloid-related gene product of bacteriophage λAPCP 168i4 deposited as ATCC40347.

6. A recombinant β-amyloid-related protein in isolated, purified form obtained by the expression of the DNA of claim 4 or 5.

7. A recombinant β-amyloid-related protein in isolated, purified form having the amino acid sequence

Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile

8. A protein according to claim 6 or 7 for use in a method of treatment of the human or animal body by diagnosis or therapy.

9. Use of a protein according to claim 6 or 7 in the manufacture of a composition useful for treating Alzheimer's disease.

10. A method of diagnosing a genetic predisposition to Alzheimer's disease in a test subject, comprising identifying, as being associated with predisposition to Alzheimer's disease, one or more alterations in the DNA of claims 1 or 2, and assaying test subject gene fragments in vitro for the presence or absence of such alteration(s).

11. A method of diagnosing a genetic predisposition to Alzheimer's disease in a test subject, comprising identifying, as being associated with a predisposition to Alzheimer's disease, one or more restriction site alterations in the DNA of claims 1, 2 or 3, and assaying test subject gene fragments in vitro for the presence or absence of such restriction site alteration(s).

12. A method of screening for Alzheimer's disease in a test subject, comprising
preparing a peptide which includes an immunogenic region of the protein of claim 6 or 7, eliciting antibodies which are specific against the peptide, and
using the antibodies to detect the increase or decrease of β-amyloid-related proteins in a sample removed from a test subject suspected of having Alzheimer's disease.

13. Use of a polypeptide of the sequence 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 for the manufacture of a composition useful for treating Alzheimer's disease.

14. A pharmaceutical composition comprising a protein according to claim 6 or 7 in admixture with at least one pharmaceutically acceptable excipient.

15. A composition for use in treatment of Alzheimer's disease which comprises a polypeptide of the sequence 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 as an active ingredient in admixture with at least one pharmaceutically acceptable excipient.

## Claims (Claims for the following Contracting State(s): ES, GR)

1. A method of diagnosing a genetic predisposition to Alzheimer's disease in a test subject, comprising:
identifying, as being associated with predisposition to Alzheimer's disease, one or more alterations in a DNA sequence comprising the DNA sequence of Figure 1 or a subfragment thereof provided that such a subfragment does not consist of DNA encoding the 28 amino-terminal amino acid residues of the β-amyloid core protein or a subfragment thereof and such a subfragment contains the sequence encoding amino acids numbers 289-345 and;
assaying test subject gene fragments in vitro for the presence or absence of such alteration(s).

2. A method according to claim 1 in which the DNA sequence comprises the 168 base pair insert fragment of the β-amyloid-related gene product of bacteriophage λAPCP168i4, deposited as ATCC 40347.

3. A method according to claim 1 or 2 comprising identifying, as being associated with a predisposition to Alzheimer's disease, one or more restriction site alterations in the DNA sequence and assaying test subject gene fragments for the presence or absence of such restriction site alteration(s).

4. A method according to claim 3 wherein the subfragment corresponds to a naturally occurring restriction fragment.

5. A method of screening for Alzheimer's disease in a test subject, comprising
preparing a peptide which includes an immunogenic region of a β-amyloid-related protein having the amino acid sequence: 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile;
 eliciting antibodies which are specific against the peptide, and
using the antibodies to detect the increase or decrease of β-amyloid-related proteins in a sample removed from a test subject suspected of having Alzheimer's disease.

6. Use of a polypeptide of the sequence 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 for the manufacture of a composition useful for treating Alzheimer's disease.

7. A process for producing a recombinant β-amyloid-related protein in isolated, purified form having the amino acid sequence 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 which process comprises expressing a DNA sequence encoding the amino acid sequence of the protein in a host cell and purifying the expressed recombinant protein.

8. A process according to claim 7 in which the DNA encoding the protein comprises the DNA sequence of Figure 1 or a subfragment thereof provided that such a subfragment does not consist of DNA encoding the 28 amino-terminal amino acid residues of the β-amyloid core protein or a subfragment thereof and such a subfragment contains the sequence encoding amino acids numbers 289-345.

9. A process according to claim 8 in which the DNA encoding the protein comprises the 168 base pair insert bacteriophage λAPCP168i4 deposited as ATCC40347.

10. A process for producing a pharmaceutical composition which comprises admixing a protein produced according to claim 7, 8 or 9 with at least one pharmaceutically acceptable excipient.

11. A process for producing a composition for use in treatment of Alzheimer's disease which process comprises admixing a polypeptide of the sequence 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 as an active ingredient with at least one pharmaceutically acceptable excipient.

12. A DNA sequence useful in the prognosis and diagnosis of Alzheimer's disease in human subjects comprising the DNA sequence of Figure 1 or a subfragment thereof provided that such a subfragment does not consist of DNA encoding the 28 amino-terminal amino acid residues of the β-amyloid core protein or a subfragment thereof and such a subfragment contains the sequence encoding amino acids numbers 289-345.

13. DNA according to claim 12 wherein the subfragment corresponds to a naturally occurring restriction fragment.

14. DNA according to claim 12 wherein DNA encodes a β-amyloid-related protein having the amino acid sequence:

Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile

15. DNA according to claim 12 or 14, which comprises the 168 base pair insert fragment of the β-amyloid-related gene product of bacteriophage AAPCP 168i4 deposited as ATCC40347.

16. A recombinant β-amyloid-related protein in isolated, purified form obtained by the expression of the DNA of claim 14 or 15.

17. A recombinant β-amyloid-related protein in isolated, purified form having the amino acid sequence

Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile

18. A protein according to claim 16 or 17 for use in a method of treatment of the human or animal body by diagnosis or therapy.

19. A pharmaceutical composition comprising a protein according to claim 16 or 17 in admixture with at least one pharmaceutically acceptable excipient.

20. A composition for use in treatment of Alzheimer's disease which comprises a polypeptide of the sequence 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 as an active ingredient in admixture with at least one pharmaceutically acceptable excipient.

## Patentansprüche (Patentansprüche für folgende(n) Vertragsstaat(en): AT, BE, CH, LI, DE, FR, GB, IT, LU, NL, SE)

1. DNA-Sequenz zur Prognose und Diagnose von Alzheimer-Krankheit bei Menschen, dadurch gekennzeichnet, daß sie die DNA-Sequenz der Figur 1 oder ein Subfragment davon umfaßt, unter der Voraussetzung, daß ein solches Subfragment nicht aus DNA besteht, die die 28 Amino-terminalen Aminosäurereste des β-Amyloid-Kernproteins oder ein Subfragment davon kodiert, und ein solches Subfragment die die Aminosäurezahlen 289-345 kodierende Sequenz enthält.

2. DNA nach Anspruch 1, dadurch gekennzeichnet, daß sie das 168 Basenpaar-Insertionsfragment des β-Amyloid-verwandten Genproduktes von Bakteriophag λAPCP168i4, hinterlegt als ATCC 40347, umfaßt.

3. DNA nach Anspruch 1 oder 2, dadurch gekennzeichnet, daß das Subfragment einem natürlich auftretenden Restriktionsfragment entspricht.

4. DNA nach Anspruch 1, dadurch gekennzeichnet, daß sie ein Subfragment mit der Aminosäuresequenz kodiert:

Glu Val Cys Ser Glu Gln Ala Glu Thr Gly Pro Cys Arg Ala
Met Ile Ser Arg Trp Tyr Phe Asp Val Thr Glu Gly Lys Cys

Ala Pro Phe Phe Tyr Gly Gly Cys Gly Gly Asn Arg Asn Asn
Phe Asp Thr Glu Glu Tyr Cys Met Ala Val Cys Gly Ser Ala
Ile.

5. DNA nach Anspruch 4, dadurch gekennzeichnet, daß sie die Sequenz des 168-Basenpaar-Insertionsfragmentes des β-Amyloid-verwandten Genproduktes von Bakteriophag λAPCP 168i4, hinterlegt als ATCC40347, umfaßt.

6. Rekombinantes β-Amyloid-verwandtes Protein in isolierter gereinigter Form, erhalten durch Expression der DNA in Anspruch 4 oder 5.

7. Rekombinantes β-Amyloid-verwandtes Protein in isolierter gereinigter Form mit der Aminosäuresequenz

Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile.

8. Protein nach Anspruch 6 oder 7 zur Verwendung in einem Verfahren zur Behandlung des menschlischen oder tierischen Körpers durch Diagnose oder Therapie.

9. Verwendung eines Proteins nach Anspruch 6 oder 7 zur Herstellung einer Zusammensetzung zur Behandlung von Alzheimer-Krankheit.

10. Verfahren zur Diagnose einer genetischen Veranlagung zur Alzheimer-Krankheit bei einer Testperson, dadurch gekennzeichnet, daß es umfaßt die Identifizierung von einer oder mehreren Veränderungen in der DNA der Ansprüche 1 oder 2, die mit der Veranlagung zur Alzheimer-Krankheit verbunden sind, und Bestimmen von Genfragmenten der Testperson in vitro auf die Anwesenheit oder Abwesenheit solcher Veränderungen.

11. Verfahren zur Diagnose einer genetischen Veranlagung zur Alzheimer-Krankheit bei einer Testperson, dadurch gekennzeichnet, daß es umfaßt die Identifizierung einer oder mehrerer Restriktionsstellen-Veränderungen in der DNA der Ansprüche 1, 2 oder 3, die mit der Veranlagung zur Alzheimer-Krankheit verbunden sind, und Bestimmen von Genfragmenten der Testperson in vitro auf die Gegenwart oder Abwesenheit solcher Restriktionsstellen-veränderungen.

12. Verfahren zum Screenen auf Alzheimer-Krankheit bei einer Testperson, dadurch gekennzeichnet, daß es umfaßt
Darstellen eines Peptids, das eine immunogene Region des Proteins des Anspruchs 6 oder 7 umfaßt, Hervorrufen von Antikörpern, die gegen das Peptid spezifisch sind, und
Verwenden der Antikörper, um die Erhöhung oder Verringerung von β-Amyloid-verwandten Proteinen in einer Probe die einer Testperson, bei der Verdacht auf Alzheimer-Krankheit besteht, entnommen wurde, zu bestimmen.

13. Verwendung eines Polypeptids der Sequenz 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
 
Phe Asp Thr Glu Glu Tyr Cys Met Ala Val Cys Gly Ser Ala
Ile
 zur Herstellung einer Zusammensetzung zur Behandlung von Alzheimer-Krankheit.

14. Pharmazeutische Zusammensetzung umfassend ein Protein nach Anspruch 6 oder 7 in Mischung mit mindestens einem pharmazeutisch annehmbaren Träger.

15. Zusammensetzung zur Behandlung von Alzheimer-Krankheit, die ein Polypeptid der Sequenz 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 als aktiven Bestandteil in Mischung mit mindestens einem pharmazeutisch annehmbaren Träger umfaßt.

## Patentansprüche (Patentansprüche für folgende(n) Vertragsstaat(en): ES, GR)

1. Verfahren zur Diagnose einer genetischen Veranlagung zur Alzheimer-Krankheit in einer Testperson, das dadurch gekennzeichnet ist, daß es umfaßt:
Identifizieren einer oder mehrerer Veränderungen, die mit der Veranlagung zur Alzheimer-Krankheit verbunden sind, in einer DNA-Sequenz, die die DNA-Sequenz der Figur 1 oder ein Subfragment davon umfaßt, unter der Voraussetzung, daß ein solches Subfragment nicht aus DNA besteht, die die 28 Amino-terminalen Aminosäurereste des β-Amyloid-Kernproteins oder ein Subfragment davon kodiert, und ein solches Subfragment die die Aminosäurezahlen 289-345 kodierende Sequenz enthält, und
Bestimmen von Genfragmenten der Testperson in vitro auf die Gegenwart oder Abwesenheit solcher Veränderungen.

2. Verfahren nach Anspruch 1, dadurch gekennzeichnet, daß die DNA-Sequenz das 168-Basenpaar-Insertionsfragment des β-Amyloid-verwandten Genprodukts von Bakteriophag λAPCP168i4, hinterlegt als ATCC 40347, umfaßt.

3. Verfahren nach Anspruch 1 oder 2, dadurch gekennzeichnet, daß es das Identifizieren einer oder mehrerer Restriktionsstellen-Veränderungen, die mit der Veranlagung zur Alzheimer-Krankheit verbunden sind, in der DNA-Sequenz und das Bestimmen von Genfragmenten der Testperson auf die Gegenwart oder Abwesenheit solcher Restriktionsstellen-Veränderungen umfaßt.

4. Verfahren nach Anspruch 3, dadurch gekennzeichnet, daß das Subfragment dem natürlich vorkommenden Restriktionstfragment entspricht.

5. Verfahren zum Screenen auf Alzheimer-Krankheit in einer Testperson, dadurch gekennzeichnet, daß es umfaßt:
Darstellen eines Peptids, das eine immunogene Region eines β-Amyloid-verwandten Proteins mit der Aminosäuresequenz: 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 und Ausbilden von Antikörpern, die spezifisch gegen dieses Peptid sind, und
die Verwendung dieser Antikörper zur Bestimmung der Erhöhung oder Verringerung von β-Amyloid-verwandten Proteinen in einer einer Testperson, bei der Verdacht auf Alzheimer-Krankheit besteht, entnommenen Probe, umfaßt.

6. Verwendung eines Polypeptids der Sequenz 
Glu Val Cys Ser Glu Gln Ala Glu Thr Gly Pro Cys Arg Ala
Met Ile Ser Arg Trp Tyr Phe Asp Val Thr Glu Gly Lys Cys
 
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 zur Herstellung einer Zusammensetzung zur Behandlung von Alzheimer-Krankheit.

7. Verfahren zur Herstellung eines rekombinanten β-Amyloid-verwandten Proteins in isolierter, gereinigter Form mit der Aminosäuresequenz 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 dadurch gekennzeichnet, daß das Verfahren die Expression einer DNA-Sequenz, die die Aminosäuresequenz des Proteins in einer Wirtszelle kodiert, und das Reinigen des exprimierten rekombinanten Proteins umfaßt.

8. Verfahren nach Anspruch 7, dadurch gekennzeichnet, daß die Protein-kodierende DNA die DNA-Sequenz der Figur 1 oder ein Subfragment davon umfaßt, vorausgesetzt, daß ein solches Subfragment nicht aus DNA besteht, die die 28 Amino-terminalen Aminosäurereste des β-Amyloid-Kernproteins oder ein Subfragment davon kodiert, und ein solches Subfragment die die Aminosäurezahlen 289-345 kodierende Sequenz enthält.

9. Verfahren nach Anspruch 8, dadurch gekennzeichnet, daß die das Protein-kodierende DNA den Bakteriophag λAPCP168i4 mit der 168-Basenpaar-Insertion, hinterlegt als ATCC40347, umfaßt.

10. Verfahren zur Herstellung einer pharmazeutischen Zusammensetzung, dadurch gekennzeichnet, daß man ein nach Anspruch 7, 8 oder 9 hergestelltes Protein mit mindestens einem pharmazeutisch annehmbaren Träger mischt.

11. Verfahren zur Herstellung einer Zusammensetzung zur Behandlung von Alzheimer-Krankheit, dadurch gekennzeichnet, daß man ein Polypeptid der Sequenz 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 als aktiven Bestandteil mit mindestens einem pharmazeutisch annehmbaren Träger mischt.

12. DNA-Sequenz zur Prognose und Diagnose von Alzheimer-Krankheit bei Menschen, dadurch gekennzeichnet, daß sie die DNA-Sequenz der Figur 1 oder ein Subfragment davon umfaßt, vorausgesetzt, daß ein solches Subfragment nicht aus DNA besteht, die die 28 Amino-terminalen Aminosäurereste des β-Amyloid-Kernproteins oder ein Subfragment davon kodiert, und ein solches Subfragment die die Aminosäurezahlen 289-345 kodierende Sequenz enthält.

13. DNA nach Anspruch 12, dadurch gekennzeichet, daß das Subfragment dem natürlich vorkommenden Restriktionsfragment entspricht.

14. DNA nach Anpsruch 12, dadurch gekennzeichnet, daß die DNA ein β-Amyloid-verwandtes Protein mit der Aminosäuresequenz: 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 kodiert.

15. DNA nach Anspruch 12 oder 14, dadurch gekennzeichnet, daß sie das 168-Basenpaar-Insertionsfragment des β-Amyloid-verwandten Genproduktes von Bakteriophag λAPCP 168i4, hinterlegt als ATCC40347, umfaßt.

16. Rekombinantes β-Amyloid-verwandtes Protein in isolierter gereinigter Form, erhalten durch Expression der DNA in Anspruch 14 oder 15.

17. Rekombinantes β-Amyloid-verwandtes Protein in isolierter gereinigter Form mit der Aminosäuresequenz

Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile.

18. Protein nach Anspruch 16 oder 17 zur Verwendung in einem Verfahren zur Behandlung des menschlischen oder tierischen Körpers durch Diagnose oder Therapie.

19. Pharmazeutische Zusammensetzung umfassend ein Protein nach Anspruch 16 oder 17 in Mischung mit mindestens einem pharmazeutisch annehmbaren Träger.

20. Zusammensetzung zur Behandlung von Alzheimer-Krankheit, die ein Polypeptid der Sequenz 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 als aktiven Bestandteil in Mischung mit mindestens einem pharmazeutisch annehmbaren Träger umfaßt.

## Revendications (Revendications pour l'(les) Etat(s) contractant(s) suivant(s): AT, BE, CH, LI, DE, FR, GB, IT, LU, NL, SE)

1. Une séquence d'ADN utile dans le pronostic et le diagnostic de la maladie d'Alzheimer chez des sujets humains, comprenant la séquence d'ADN de la Figure 1 ou l'un de ses sous-fragments, à condition que ce sous-fragment ne soit pas constitué d'ADN codant pour les 28 résidus d'acide aminé amino-terminaux de la protéine centrale β-amyloïde ou de l'un de ses sous-fragments et que ce sous-fragment contienne la séquence codant pour les acides aminés numéros 289-345.

2. ADN selon la revendication 1, qui comprend le fragment d'insert de 168 paires de bases du produit de gène apparenté à la β-amyloïde du bactériophage λAPCP168i4, déposé sous ATCC 40347.

3. ADN selon la revendication 1 ou 2, où le sous-fragment correspond à un fragment de restriction naturel.

4. ADN selon la revendication 1 qui code pour un sous-fragment ayant la séquence d'acides aminés :

Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile

5. ADN selon la revendication 4, ayant la séquence du fragment d'insert de 168 paires de bases du produit de gène apparenté à la β-amyloïde du bactériophage λAPCP 168i4, déposé sous ATCC40347.

6. Une protéine recombinante apparentée à la β-amyloïde sous forme isolée, purifiée, obtenue par l'expression de l'ADN de la revendication 4 ou 5.

7. Une protéine recombinante apparentée à la β-amyloïde sous forme isolée, purifiée, ayant la séquence d'acides aminés

Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile

8. Protéine selon la revendication 6 ou 7 destinée à être utilisée dans une méthode de traitement de l'organisme humain ou animal par diagnostic ou thérapie.

9. Utilisation d'une protéine selon la revendication 6 ou 7 dans la fabrication d'une composition utile pour le traitement de la maladie d'Alzheimer.

10. Une méthode de diagnostic d'une prédisposition génétique à la maladie d'Alzheimer chez un sujet à tester, comprenant l'identification, comme étant associées à une prédisposition à la maladie Alzheimer, d'une ou plusieurs altérations dans la séquence d'ADN des revendications 1 ou 2 et le test in vitro de la présence ou de l'absence de cette ou ces altérations dans des fragments de gène de sujet à tester.

11. Une méthode de diagnostic d'une prédisposition génétique à la maladie d'Alzheimer chez un sujet à tester, comprenant l'identification, comme étant associées à une prédisposition à la maladie Alzheimer, d'une ou plusieurs altérations de sites de restriction dans l'ADN des revendications 1, 2 ou 3, et le test in vitro de la présence ou de l'absence de cette ou ces altérations de sites de restriction dans des fragments de gène de sujet à tester.

12. Une méthode de criblage de la maladie d'Alzheimer chez un sujet à tester, comprenant
la préparation d'un peptide qui inclut une région immunogène de la protéine de la revendication 6 ou 7, la production d'anticorps qui sont spécifiques du peptide, et
l'utilisation des anticorps pour détecter l'augmentation ou la diminution de la quantité de protéines apparentées à la β-amyloïde dans un échantillon prélevé chez un sujet à tester suspecté d'être atteint de la maladie d'Alzheimer.

13. Utilisation d'un polypeptide de séquence 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 pour la fabrication d'une composition utile pour le traitement de la maladie d'Alzheimer.

14. Une composition pharmaceutique comprenant une protéine selon la revendication 6 ou 7 en mélange avec au moins un excipient pharmaceutiquement acceptable.

15. Une composition destinée à être utilisée dans le traitement de la maladie d'Alzheimer, qui comprend un polypeptide de séquence 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 en tant qu'ingrédient actif en mélange avec au moins un excipient pharmaceutiquement acceptable.

## Revendications (Revendications pour l'(les) Etat(s) contractant(s) suivant(s): ES, GR)

1. Une méthode de diagnostic d'une prédisposition génétique à la maladie d'Alzheimer chez un sujet à tester, comprenant :
l'identification, comme étant associées à une prédisposition à la maladie Alzheimer, d'une ou plusieurs altérations dans une séquence d'ADN comprenant la séquence d'ADN de la Figure 1 ou de l'un de ses sous-fragments, à condition que ce sous-fragment ne soit pas constitué d'ADN codant pour les 28 résidus d'acide aminé amino-terminaux de la protéine centrale β-amyloïde ou de l'un de ses sous-fragments et que ce sous-fragment contienne la séquence codant pour les acides aminés numéros 289-345 et ;
le test in vitro de la présence ou de l'absence de cette ou ces altérations dans des fragments de gène de sujet à tester.

2. Méthode selon la revendication 1, dans laquelle la séquence d'ADN comprend le fragment d'insert de 168 paires de bases du produit de gène apparenté à la β-amyloïde du bactériophage λAPCP168i4, déposé sous ATCC 40347.

3. Méthode selon la revendication 1 ou 2, comprenant l'identification, comme étant associées à une prédisposition à la maladie Alzheimer, d'une ou plusieurs altérations de sites de restriction dans la séquence d'ADN, et le test in vitro de la présence ou de l'absence de cette ou ces altérations de sites de restriction dans des fragments de gène de sujet à tester.

4. Méthode selon la revendication 3, dans laquelle le sous-fragment correspond à un fragment de restriction naturel.

5. Une méthode de criblage de la maladie d'Alzheimer chez un sujet à tester, comprenant
la préparation d'un peptide qui inclut une région immunogène d'une protéine apparentée à la β-amyloïde ayant la séquence d'acides aminés : 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile;
 la production d'anticorps qui sont spécifiques du peptide, et
l'utilisation des anticorps pour détecter l'augmentation ou la diminution de la quantité de protéines apparentées à la β-amyloïde dans un échantillon prélevé chez un sujet à tester suspecté d'être atteint de la maladie d'Alzheimer.

6. Utilisation d'un polypeptide de séquence 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 pour la fabrication d'une composition utile pour le traitement de la maladie d'Alzheimer.

7. Un procédé de production d'une protéine recombinante apparentée à la β-amyloïde sous une forme isolée, purifiée, ayant la séquence d'acides aminés 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 ce procédé comprenant l'expression d'une séquence d'ADN codant pour la séquence d'acides aminés de la protéine dans une cellule hôte et la purification de la protéine recombinante exprimée.

8. Procédé selon la revendication 7, dans lequel l'ADN codant pour la protéine comprend la séquence d'ADN de la Figure 1 ou l'un de ses sous-fragments, à condition que ce sous-fragment ne soit pas constitué d'ADN codant pour les 28 résidus d'acide aminé amino-terminaux de la protéine centrale β-amyloïde ou de l'un de ses sous-fragment et que ce sous-fragment contienne la séquence codant pour les acides aminés numéros 289-345.

9. Procédé selon la revendication 8, dans lequel l'ADN codant pour la protéine comprend le bactériophage insert de 168 paires de bases λAPCP168i4, déposé sous ATCC40347.

10. Un procédé de production d'une composition pharmaceutique qui comprend le mélange d'une protéine produite selon la revendication 7, 8 ou 9 avec au moins un excipient pharmaceutiquement acceptable.

11. Un procédé de production d'une composition pour une utilisation dans le traitement de la maladie d'Alzheimer, ce procédé comprenant le mélange d'un polypeptide de séquence 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 en tant qu'ingrédient actif avec au moins un excipient pharmaceutiquement acceptable.

12. Une séquence d'ADN utile dans le pronostic et le diagnostic de la maladie d'Alzheimer chez des sujets humains, comprenant la séquence d'ADN de la Figure 1 ou de l'un de ses sous-fragments, à condition que ce sous-fragment ne soit pas constitué d'ADN codant pour les 28 résidus d'acide aminé amino-terminaux de la protéine centrale β-amyloïde ou de l'un de ses sous-fragments et que ce sous-fragment contienne la séquence codant pour les acides aminés numéros 289-345.

13. ADN selon la revendication 12, où le sous-fragment correspond à un fragment de restriction naturel.

14. ADN selon la revendication 12 où l'ADN code pour une protéine apparentée à la β-amyloïde ayant la séquence d'acides aminés :

Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile

15. ADN selon la revendication 12 ou 14, qui comprend le fragment d'insert de 168 paires de bases du produit de gène apparenté à la β-amyloïde du bactériophage λAPCP 168i4, déposé sous ATCC40347.

16. Une protéine recombinante apparentée à la β-amyloïde sous forme isolée, purifiée, obtenue par l'expression de l'ADN de la revendication 14 ou 15.

17. Une protéine apparentée à la β-amyloïde sous forme isolée, purifiée, ayant la séquence d'acides aminés

Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile

18. Protéine selon la revendication 16 ou 17 destinée à être utilisée dans une méthode de traitement de l'organisme humain ou animal par diagnostic ou thérapie.

19. Une composition pharmaceutique comprenant une protéine selon la revendication 16 ou 17 en mélange avec au moins un excipient pharmaceutiquement acceptable.

20. Une composition pour une utilisation dans le traitement de la maladie d'Alzheimer qui comprend un polypeptide de séquence 
Glu Val Cys Ser Glu Gln Ala Glu Thr Gly Pro Cys Arg Ala
Met Ile Ser Arg Trp Tyr Phe Asp Val Thr Glu Gly Lys Cys
Ala Pro Phe Phe Tyr Gly Gly Cys Gly Gly Asn Arg Asn Asn
Phe Asp Thr Glu Glu Tyr Cys Met Ala Val Cys Gly Ser Ala
Ile
 en tant qu'ingrédient actif en mélange avec au moins un excipient pharmaceutiquement acceptable.
